# FASCICULE DE BREVET EUROPEEN

(11) **EP 2 313 401 B1**
(45) Date de publication et mention de la délivrance du brevet: **11.11.2015**
(21) Numéro de dépôt: 09784508.5
(22) Date de dépôt: 10.07.2009
(51) Int. Cl.: C07D 413/10, C07D 263/32, C07D 233/64, G01T 1/02

(54) **MOLÉCULES POLYCYCLIQUES DISCRIMINANTES POUR LES RAYONNEMENTS NEUTRON ET GAMMA**
POLYZYKLISCHE MOLEKÜLE, DIE ZWISCHEN NEUTRONEN- UND GAMMASTRAHLUNG UNTERSCHEIDEN
POLYCYCLIC MOLECULES DISCRIMINATING BETWEEN NEUTRON AND GAMMA RAYS

(30) Priorité: 11.07.2008 FR 0854740
(43) Date de publication de la demande: 27.04.2011
(73) Titulaire: Centre National de la Recherche Scientifique (CNRS), 75016 Paris (FR); Université de Strasbourg, 67081 Strasbourg Cedex (FR)
(72) Inventeur: BARILLON, Rémi, F-67520 Marlenheim (FR); BOUAJILA, Ezeddine, F-69100 Villeurbanne (FR); DOUCE, Laurent, F-67100 Strasbourg (FR); JUNG, Jean-Marc, F-67270 Gougenheim (FR); STUTTGE, Louise, F-67000 Strasbourg (FR)
(74) Mandataire: Blot, Philippe Robert Emile
(86) Numéro de dépôt international: PCT/FR2009/051382
(87) Numéro de publication internationale: WO 2010/004228

(56) Documents cités:
- MEYER H-J ET AL: "Water-binding solid scintillators: synthesis, emission properties, and tests in 3H and 14C counting" CHEMISTRY - A EUROPEAN JOURNAL, WILEY - V C H VERLAG GMBH & CO. KGAA, WEINHEIM, DE, vol. 6, no. 15, 1 janvier 2000 (2000-01-01), pages 2809-2817, XP002248372 ISSN: 0947-6539
- MCCAIRN M C ET AL: "Synthesis, evaluation and incorporation into liposomes of 4-functionalised-2,5-diphenyloxazole derivatives for application in scintillation proximity assays" TETRAHEDRON LETTERS, ELSEVIER, AMSTERDAM, vol. 45, no. 10, 1 mars 2004 (2004-03-01), pages 2163-2166, XP004489980 ISSN: 0040-4039
- A. R. KATRITZKY ET AL.: "Synthesis of Fluorescent and Coloured Pyrylium and Pyridinium Salts" JOURNAL OF HETEROCYCLIC CHEMISTRY, vol. 21, 1984, pages 1673-1677, XP002518336
- BROOKS F D: "DEVELOPMENT OF ORGANIC SCINTILLATORS", NUCLEAR INSTRUMENTS AND METHODS,, vol. 162, 1 January 1979 (1979-01-01), pages 477-505, XP001431823,

## Description

La présente invention concerne une nouvelle famille de molécules discriminantes pour les rayonnements neutron et gamma notamment et leur procédé de préparation. Ces molécules peuvent aussi être utilisées pour la détection des rayonnements nucléaires, en recherche fondamentale ou bien dans les domaines de la dosimétrie et de l'imagerie médicales.

La production de paires électron-trou par ionisation constitue la première étape, l'une des plus rapides, de la relaxation de l'énergie initialement déposée par un rayonnement dans un milieu. Dans la matière organique excitée, les paires formées sont généralement constituées d'un électron quasi-libre dans la bande de conduction, géminé (électriquement apparié) à un trou beaucoup moins mobile dans la bande de valence.

La discrimination repose sur le fait que la quantité d'énergie, dE, déposée dans un milieu par unité de distance parcourue, dx, par un rayonnement incident, est différente selon que ce rayonnement soit formé de photons ou bien de particules chargées. Dans le cas d'une excitation par des photons gammas issus, par exemple, d'une source radioactive, la quantité dE/dx est relativement faible, ce qui implique de faibles densités d'ionisation, et donc une production de paires électron-trou, relativement faible. Dans le cas d'une excitation par des neutrons, il se produit différentes réactions nucléaires, dont la plus importante est la réaction (n,p) au cours de laquelle un proton (p) est éjecté d'un noyau par collision élastique avec un neutron (n) incident. Ce proton va naturellement ralentir dans le milieu par excitation et ionisation de ce dernier, produisant des paires électron-trou géminées de même nature que celles produites avec un rayonnement gamma, mais avec une densité d'ionisation bien plus forte.

La recombinaison différée des paires produites étant à l'origine de l'émission de fluorescence, cette dernière sera plus intense lors d'une excitation par des neutrons (indirectement par des protons) que par des photon gammas. La comparaison des intensités de fluorescence émises lors des deux types d'excitation, photons et neutrons, permet donc la discrimination.

La discrimination des rayonnements neutron et gamma est généralement réalisée selon trois types de méthodes :
- par mesure de temps de vol des particules gamma et des neutrons ;
- par l'utilisation de détecteurs à base de semi-conducteurs ; ou
- par l'utilisation de molécules scintillantes diluées au sein de matériaux plastiques transparents.

La méthode qui consiste à faire des mesures de temps de vol est particulièrement complexe de mise en oeuvre sur les plans technique et opératoire, notamment en raison de la difficulté d'accéder à une bonne référence temporelle. Elle nécessite, en effet, une technicité importante, tant dans la fabrication matérielle de l'expérience que dans sa conduite. Cette technique, qui donne cependant des résultats très précis, reste essentiellement dédiée aux laboratoires de recherche et aux applications en milieu peu hostile.

La méthode qui consiste à utiliser comme partie active d'un détecteur un matériau semi-conducteur reste limitée aux fortes fluences. Dans ce domaine d'application, la principale limitation à l'utilisation de matériaux conducteurs réside toujours dans le fait que la mesure se fait sur la quantité de charges produites ou bien le courant généré. Dans tous les cas, les mesures restent peu sensibles car la limite de détection d'un courant se situe aux environs du picoampère et correspond donc qu'à la production d'environ 10 millions de porteurs de charges mobiles chaque seconde. On compense généralement la faible sensibilité de cette méthode par l'augmentation du volume sensible du détecteur, mais se posent alors de nouveaux problèmes, liés à la diminution globale de la conduction de l'ensemble ainsi qu'à la dégradation de la réponse temporelle du détecteur.

Les mesures basées sur la scintillation de molécules diluées sont de loin les plus utilisées.

Ainsi, lorsque le milieu irradié contient des molécules luminescentes, la recombinaison géminée des paires électron-trou résultant de l'ionisation primaire conduit, avec un rendement défini, à une émission de fluorescence différée dont l'intensité sera proportionnelle à la densité des ionisations produites par le rayonnement sur son parcours. Cette intensité est plus élevée lors du passage d'un proton que lors du passage d'un photon en raison d'une perte d'énergie par unité de parcours, dE/dx, plus importante. Ainsi, par une mesure de fluorescence différée, on peut discriminer entre la fluorescence résultant du passage d'un proton [produit par un neutron lors d'une réaction (n,p)] et celle résultant du passage d'un photon. La comparaison des intensités des deux signaux de fluorescence émis permet d'effectuer la discrimination. La difficulté de la mesure résulte essentiellement dans le fait que la fluorescence n'est émise que durant un temps très court, de quelques centaines de nanosecondes, après le passage du rayonnement primaire, ce qui implique une instrumentation spécifique, fonctionnant avec une résolution temporelle nanoseconde. L'intensité de fluorescence étant globalement faible, on ne peut la détecter qu'à l'aide de photomultiplicateurs. Le spectre d'émission de la fluorescence est indépendant de la nature de l'excitation et ne dépend que de la nature du chromophore choisi. La sensibilité de détection est bien supérieure à celle que l'on peut attendre d'une mesure de courant car il suffit que quelques milliers porteurs de charges libres soient produits dans les milieux pour que la détection devienne possible.

Les molécules scintillantes diluées sont généralement des oxazoles ou des oxadiazoles, voire du para-terphényle ou de l'anthracène. Toutes ces molécules ont des propriétés de luminescence nanoseconde dans un domaine spectral s'étendant de 300 à 400 nm.

Cette méthode, basée sur la mesure de fluorescence de recombinaison, après interception des charges produites dans le milieu par les molécules scintillantes, est de loin la plus rapide (nanoseconde) et la plus sensible. En théorie, la production d'une seule charge libre dans le milieu suffit à produire une fluorescence détectable et donc une mesure. La méthode serait donc 10 millions fois plus sensible que celle basée sur la conduction. Mais, dans la pratique actuelle, le fait que la limite de dilution d'une molécule scintillante dans un plastique se situe vers 0.01 mole/L, réduit d'un facteur 10 à 100 la sensibilité. L'autre facteur de réduction de la sensibilité est donné par l'angle solide de détection de la lumière. On peut l'estimer de 100 à 1000. L'utilisation d'un photomultiplicateur ayant un rendement de détection typique de 20 % réduit donc la sensibilité d'un facteur 5 environ. Globalement, on peut estimer que les techniques de fluorescence sont environ 1000 fois plus sensibles que les techniques électriques et que la principale limitation provient de la dilution des molécules fluorescentes. L'utilisation de matériaux fluorescents aromatiques non dilués est possible, soit à l'état liquide (benzène, xylène), soit à l'état solide (p-terphényle, anthracène). Ces matériaux sont cependant hautement inflammables et toxiques. Le benzène, par exemple, et le xylène utilisé commercialement sur l'appellation Ne213, sont des agents intercalants, donc cancérigènes et mutagènes. Ils sont également irritants pour la peau et les yeux.

Il a maintenant été découvert une nouvelle famille de molécules fluorescentes permettant également la discrimination en temps réel des rayonnements neutron et gamma notamment. Ces molécules résultent de l'association d'une entité fluorescente avec une entité ionique comprenant un hétéroatome choisi parmi N, S ou P porteur d'une charge positive, par exemple une entité ionique de type imidazolium. Contre toute attente, les composés ainsi obtenus présentent d'une part, des propriétés de fluorescence rapides (nanoseconde) et d'émission dans l'ultraviolet (400 nm), et d'autre part les propriétés des liquides ioniques. Le fait que ces matériaux possèdent au moins un groupe fluorophore par molécule leur confère des rendements de fluorescence importants, semblables à celles des matériaux non dilués, mais avec de nombreuses propriétés supplémentaires :
- tension de vapeur non mesurable permettant une utilisation sous vide poussé ;
- bonne stabilité thermique (200°C) ;
- caractère ininflammable ;
- faible toxicité ;
- état liquide ou solide ;
- mise en forme et usinage plus facile ;
- propriétés cristal liquide.

Outre ces propriétés, la réalisation de molécules à façon permet d'ajuster l'efficacité d'interaction initiale avec le rayonnement afin d'augmenter la perte d'énergie (dE/dx) et donc la quantité de lumière émise. Ceci peut être obtenu, par exemple, par l'adjonction d'un atome lourd (détection des photons), ou par l'augmentation du nombre d'atome d'hydrogènes (détection des neutrons). Une meilleure sensibilité de détection est obtenue, en milieux purs et dilués, dans la mesure où l'on contrôle mieux la construction de la molécule et les paramètres d'interaction.

Ainsi, selon un premier objet, l'invention concerne l'utilisation pour la détection des rayonnements gamma, X, neutron, proton, d'un composé fluorophore porteur d'un groupe organique cationique associé à un contre-anion, ou bien porteur d'un groupe anionique associé à un contre-cation organique, ledit groupe cationique ou contre-cation organique comportant au moins un hétéroatome choisi parmi N, S ou P porteur d'une charge positive, ledit composé fluorophore répondant à la formule (la) : 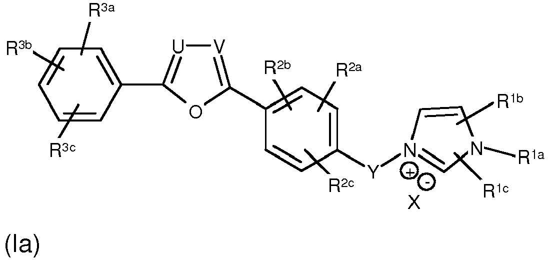 dans laquelle :
R^{1a} est un groupe alkyle en C₁-C₃₀ ;
R^{1b} et R^{1c} sont H ; 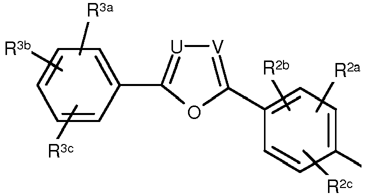 est un groupe fluorophore dans lequel :
   R^{2a}, R^{2b}, R^{2c}, R^{3a}, R^{3b}, R^{3c} sont, à chaque occurrence, chacun indépendamment choisis parmi H, les groupes alkyles en C₁-C₃₀, et cycloalkyles en C₃-C₇;
   Y est un groupe alkylène en C₁-C₆
   U, V représentent indépendamment CR²⁶ ou N, étant entendu que l'un au moins de U ou V représente N ;
   R²⁶ est H, C₁-C₆ alkyle, OR²², NR²³R²⁴, NO₂, C(=O)R²², CO₂R²², OC(=O)R²², C(=O)NR²³R²⁴, OC(=O)NR²³R²⁴ SO₃H, PO₄H, CN ;
   R²² est, à chaque occurrence, indépendamment choisi parmi H, alkyle en C₁-C₆, aryle en C₆-C₁₀ et aralkyle ;
   R²³ et R²⁴ sont, à chaque occurrence, indépendamment choisis parmi H, alkyle en C₁-C₆ et aryle en C₆-C₁₀, ou bien R²³ et R²⁴ forment avec l'atome d'azote auquel ils sont attachés un groupe hétérocyclique de 3 à 7 chaînons ;
   X- est un anion organique ou inorganique.

Le composé fluorophore (la) est porteur d'un groupe cationique organique associé à un contre-anion.

Le présent brevet décrit des composés dans lesquels le groupe organique cationique est un groupe hétérocyclique éventuellement substitué par un à trois groupes choisis parmi R^{1a}, R^{1b}, R^{1c} ou R²⁵,
ou bien le groupe organique cationique est un groupe (a), (b), (c) ou (d) : 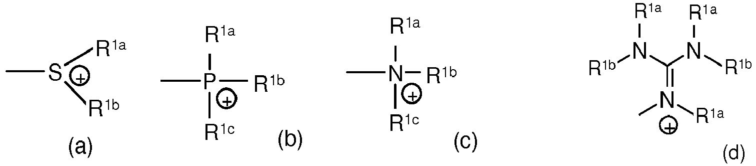 où
R^{1a}, R^{1b}, R^{1c} sont, à chaque occurrence, chacun indépendamment choisis parmi H, les groupes alkyles en C₁-C₃₀, cycloalkyles en C₃-C₇, aryle en C₆-C₁₀, hétéroaryle, aralkyle, hétéroarylalkyle, un groupe fluorophore, dans laquelle lesdits groupes alkyles ou aryles sont éventuellement substitués par 1 à 3 groupes R²⁰ ;
R²⁵ est indépendamment choisi parmi OH, NH₂, =O, C(=O)OR²¹ ;
R²⁰ est, à chaque occurrence, indépendamment choisi parmi F, Cl, Br, I, OR²², NR²³R²⁴, NHOH, NO₂, CN, CF₃, alkyle en C₁-C₆, alcényle en C₂-C₆, alcynyle en C₂-C₆, aryle en C₆-C₁₀, aralkyle, =O, C(=O)R²², CO₂R²², OC(=O)R²², C(=O)NR²³R²⁴, OC(=O)NR²³R²⁴, NR²1C(=S)R²² et S(O)_{y}R²²;
R²¹ est, à chaque occurrence, indépendamment choisi parmi H ou alkyle en C₁-C₆ ;
R²² est, à chaque occurrence, indépendamment choisi parmi H, alkyle en C₁-C₆, aryle en C₆-C₁₀ et aralkyle ;
R²³ et R²⁴ sont, à chaque occurrence, indépendamment choisis parmi H, alkyle en C₁-C₆ et aryle en C₆-C₁₀, ou bien R²³ et R²⁴ forment avec l'atome d'azote auquel ils sont attachés un groupe hétérocyclique de 3 à 7 chaînons ;

L'invention concerne l'utilisation d'un composé de formule (la) répondant à la formule (A) plus générale:

L*-Y-Z^{+ -}X

dans laquelle :
Z⁺ est un groupe organique cationique tel que défini ci-dessus,
X- est un anion organique ou inorganique,
Y est un groupe alkylène en C₁-C₆ ou
   un groupe (alkylène en C₁-C₄)ₘ-Q-(alkylène en C₁-C₄)ₙ ;
   un groupe arylène en C₆-C₁₀ ;
dans laquelle les groupes alkylène, arylène sont éventuellement substitués par 1 à 3 groupes R²⁰ ;
Q est un groupe -C(=O)-, -NR²¹C(=O)-, -C(=O)NR²¹-, -C(=O)O-, -OC(=O)-, -O-, -NR²¹-, -S(O)_{y}-, -CR²¹=CR²¹-, -C≡C-, arylène en C₆-C₁₀, hétéroarylène de 5 à 10 chaînons, cycloalkylène en C₃-C₆ ou hétérocycloalkylène de 3 à 6 chaînons,
   dans laquelle lesdits groupes arylène, hétéroarylène, cycloalkylène sont éventuellement substitués par 1 à 3 groupes R²⁰ ;
L* est un groupe fluorophore F₁,
m est 0 ou 1 ;
n est 0 ou 1 ;
y est 0, 1 ou 2,
les groupes R²⁰, et R²¹ étant tels que définis ci-dessus.

De façon privilégiée, l'invention concerne l'utilisation des fluorophores définis par la formule (la) ci-dessus pour la discrimination des rayonnements proton/gamma, proton/X, neutron/gamma, neutron/X, alpha/gamma, alpha/X.

Le groupe organique cationique est un groupe hétérocyclique comportant au moins un atome d'azote. A titre d'exemple de groupes décrit par le présent brevet, on peut citer notamment les groupes pyridile, imidazolyle, imidazolidinyle, oxazolyle, oxazolidinyle, thiazolyle, thiazolidinyle, pyrazolyle, triazolyle, tétrazolyle, benzotriazolyle, un groupe guanine ou un groupe caprolactame.

Le présent brevet décrit, un groupe organique cationique choisi parmi :

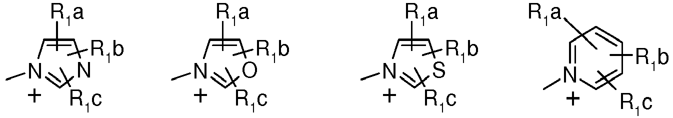

Le présent brevet décrit encore un groupe pyridile ou imidazolyle, notamment :

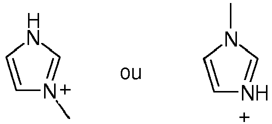

Le présent brevet décrit aussi comme composé fluorophore F₁ porteur d'un groupement cationique ou anionique un radical oxazolyle, oxadiazolyle, anthracényle, ou phénanthrényle.

Les composés de formule (la) revendiqués et utiles selon l'invention répondent aussi à la formule (I) plus générale : 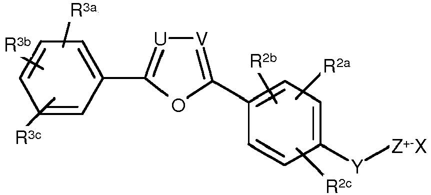 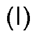 dans laquelle :
U, V représentent indépendamment CR²⁶ ou N, étant entendu que l'un au moins de U ou V représente N ;
R²⁶ est H, C₁-C₆ alkyle, OR²², NR²³R²⁴, NO₂, C(=O)R²², CO₂R²², OC(=O)R²², C(=O)NR²³R²⁴, OC(=O)NR²³R²⁴, SO₃H, PO₄H, CN.
R^{2a}, R^{2b}, R^{2c}, R^{3a}, R^{3b}, R^{3c} sont, à chaque occurrence, chacun indépendamment choisis parmi H, les groupes alkyles en C₁-C₃₀, cycloalkyles en C₃-C₇, aryle en C₆-C₁₀, hétéroaryle, aralkyle, hétéroarylalkyle, dans laquelle lesdits groupes alkyles ou aryles sont éventuellement substitués par 1 à 3 groupes R²⁰ ;
Y, Z⁺, X⁻, R²⁰, R²², R²³, R²⁴ étant tels que définis ci-dessus.

Selon un autre aspect, l'invention concerne l'utilisation d'un composé fluorophore de formule (la) tel que défini ci dessus, pour la fabrication d'un instrument de radiodétection, de dosimétrie industrielle ou médicale.

Les composés Les composés de formule (la) revendiqués répondent à la formule (I) plus générale : 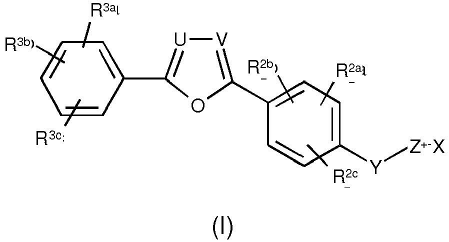 dans laquelle Z⁺, X⁻, Y, U, V, R^{2a}, R^{2b}, R^{2c}, R^{3a}, R^{3b}, R^{3c} sont tous tels que définis ci-dessus.

Les composés selon l'invention, revendiqués, répondent à la formule (la) :

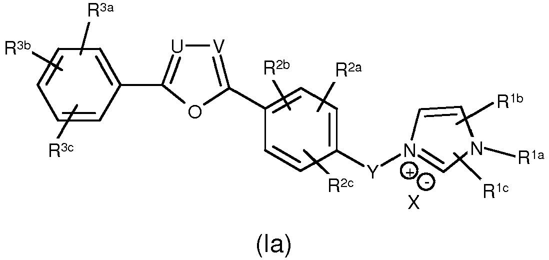

De préférence, R^{1a} est un groupe alkyle en C₁-C₁₆.

De préférence, R^{1b}, R^{1c} sont H.

De façon préférée, U est CR²⁶, et notamment CH.

Selon une variante, Y est un groupe alkylène en C₁-C₆, notamment un groupe CH₂.

De préférence, X⁻ est un anion choisi parmi un halogénure, P(R⁴)₆-, B(R⁴)4⁻, SCN⁻, (R⁵SO₂)₂N⁻, R⁵OSO₃, R⁵SO₃⁻, carborane, carbonate (CO₃²⁻), hydrogénocarbonate (HCO₃⁻), alcoolate (R⁴O⁻), carboxylate (R⁴COO⁻), amidure (NH₂⁻), phosphate (PO₄⁻), SiF₆⁻, SbF₆⁻, I₃⁻, nitrate (NO₃⁻), oxyde d'halogénure, silicate, sulfate (SO₄⁻), sulfonate (R⁴SO₃⁻), cyanure (CN⁻), carbanions, ou métallate ;
où :
R⁴ est, à chaque occurrence, un groupe indépendamment choisi parmi un atome d'halogène, un groupe alkyle en C₁-C₆, un groupe aryle en C₆-C₁₀, un groupe aralkyle ;
R⁵ est, à chaque occurrence, un groupe indépendamment choisi parmi alkyle en C₁-C₂₀, halogénoalkyle en C₁-C₂₀, aryle en C₆-C₁₀, aralkyle.

Le terme "Métallate" désigne un complexe anionique contenant un métal, notamment un métal de transition complexé par plusieurs ligands, par exemple un chalcogène tel que l'oxygène ou un groupe cyanure. De préférence, l'anion métallate est un groupe cyanométallate ou oxométallate.

Le terme "carborane" désigne une molécule constituée d'atomes de bore, de carbone et d'hydrogène et portant une charge négative. A titre d'exemple, on peut citer CB₁₁H₁₂⁻.

Le terme "oxyde d'halogénure" désigne des oxydes de formule HalOₓ⁻ où Hal représente Br, Cl ou I et x est un entier de 1 à 4. A titre d'exemple, on peut citer ClO⁻₄ , IO₃⁻.

Le terme "carbanion" désigne un composé comprenant un atome de carbone porteur d'une charge négative. A titre d'exemple, on peut citer (CF₃SO₂)₃C⁻.

Plus préférentiellement, X⁻ est choisi parmi Br⁻, PF₆⁻, BF₄⁻, (CF₃SO₂)₂N⁻, C₁₂H₂₅OSO₃⁻, C₁₆H₃₃OSO₃⁻, CF₃SO₃⁻.

Parmi les composés préférés, on peut citer notamment :
- bromure de 1-méthyle-3-[4-(5-phényl-oxazol-2-yl)-benzyl]-3H-imidazol-1-ium
- bromure de 1-hexyl-3-[4-(5-phényl-oxazol-2-yl)-benzyl]-3H-imidazol-1-ium
- bromure de 1-octyl-3-[4-(5-phényl-oxazol-2-yl)-benzyl]-3H-imidazol-1-ium
- bromure de 1-décyl-3-[4-(5-phényl-oxazol-2-yl)-benzyl]-3H-imidazol-1-ium
- bromure de 1-dodécyl-3-[4-(5-phényl-oxazol-2-yl)-benzyl]-3H-imidazol-1-ium
- bromure de 3-[4-(5-phényl-oxazol-2-yl)-benzyl]-1-tétradécyl-3H-imidazol-1-ium
- bromure de 1-hexadécyl-3-[4-(5-phényl-oxazol-2-yl)-benzyl]-3H-imidazol-1-ium
- hexafluorophosphate de 1-hexadécyl-3-[4-(5-phényl-oxazol-2-yl)-benzyl]-3H-imidazol-1-ium
- 1-hexadécyl-3-[4-(5-phényl-oxazol-2-yl)-benzyl]-3H-imidazol-1-ium bis(trifluorométhylsulfonyl)imide
- 1-hexadécyl-3-[4-(5-phényl-oxazol-2-yl)-benzyl]-3H-imidazol-1-ium dodécyl sulfate
- 1-hexadécyl-3-[4-(5-phényl-oxazol-2-yl)-benzyl]-3H-imidazol-1-ium hexadécyl sulfate.

Selon un autre objet, l'invention concerne un matériau, notamment un matériau plastique transparent comprenant un composé tel que défini ci-dessus.

Les composés selon l'invention peuvent être préparés par application ou adaptation de toute méthode connue en soi de et/ou à la portée de l'homme du métier, notamment celles décrites par Larock dans *Comprehensive Organic Transformations,* VCH Pub., 1989, ou par application ou adaptation des procédés décrits dans les exemples qui suivent.

Les composés selon l'invention peuvent être notamment préparés en faisant réagir un composé fluorophore porteur d'un groupe partant avec un groupe comportant un hétéroatome nucléophile choisi parmi N, S ou P, de manière à former un groupe organique cationique tel que défini ci-dessus.

Ainsi, selon un mode de réalisation particulier, l'invention concerne un procédé de préparation d'un composé de formule (la) comprenant la réaction d'un composé de formule (II) avec un composé de formule (III) : 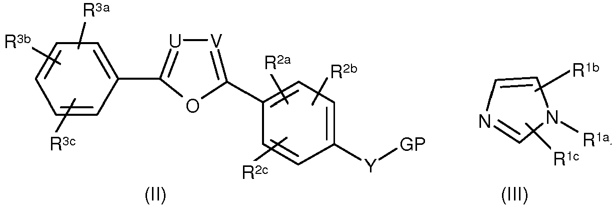 dans lesquelles R^{1a}, R^{1b}, R^{1c}, R^{2a}, R^{2b}, R^{2c}, R^{3a}, R^{3b}, R^{3c}, U, et Y sont tels que définis ci-dessus et GP représente un groupe partant.

Par « groupe partant », on entend un groupe chimique labile, i.e pouvant être facilement substitué par un groupe nucléophile. Comme exemple de groupe partant, on peut citer notamment Cl, Br, I ou les sulfonates tels que mésylate ou tosylate.

Eventuellement, ledit procédé peut également comprendre l'étape consistant à isoler le produit obtenu.

Cette réaction consiste généralement en une substitution d'ordre 2 des précurseurs de formules (II) et (III). Cette réaction est généralement réalisée dans un solvant polaire aprotique, notamment dans un éther tel que le tétrahydrofurane. Les composés de formules générales (II) et (III) peuvent être préparés par application ou adaptation de toute méthode connue en soi et/ou à la portée de l'homme du métier, plus particulièrement selon la méthode décrite dans la littérature [1] ou dans [2 à 5] pour les composés de formule (III)).

Les composés selon l'invention peuvent être également préparés à partir d'un composé fluorophore porteur d'un groupe précurseur d'un groupe cationique tel que défini ci-dessus, plus précisément un groupe non chargé électroniquement comportant un hétéroatome choisi parmi N, S ou P. Plus particulièrement, les composés selon l'invention peuvent être préparés en faisant réagir un tel composé avec un composé électrophile, par exemple avec un acide ou un agent alkylant.

A titre illustratif et non limitatif de cette voie de synthèse, on peut citer le schéma réactionnel ci-après.

| | |
|---|---|
| 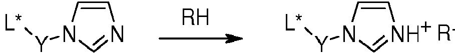 | RH = acide faible tel que RCO₂H |
| 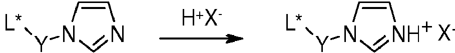 | H⁺X⁻ = acide fort tel que HCl |
| 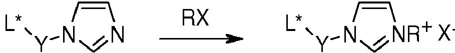 | RX = agent alkylant tel que Mel |
| L*, Y étant tels que définis ci-dessus | |

Les composés selon l'invention peuvent être également préparés en introduisant sur le composé fluorophore, un groupe anionique, par exemple un anion carboxylate (CO₂⁻) et en associant cet anion à un contre-cation organique comportant un hétéroatome choisi parmi N, S ou P, ledit hétéroatome étant porteur de la charge positive.

Le schéma réactionnel ci-dessous est donné à titre illustratif et non limitatif de cette voie de synthèse. 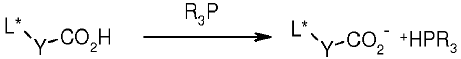 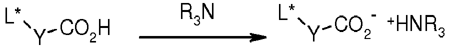 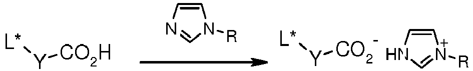 L*, Y étant tels que définis ci-dessus

### DEFINITIONS

Tels qu'on les utilise ci-dessus et dans toute la description de l'invention, les termes suivants, sauf mention contraire, doivent être compris comme ayant les significations suivantes.

Selon la présente invention, le terme « fluorophore » désigne un groupe fluorescent, capable d'absorber de l'énergie à une longueur d'onde spécifique et de réémettre de l'énergie à une longueur d'onde différente mais également spécifique. La quantité et la longueur d'onde de l'énergie réémise dépendent à la fois du fluorophore et de l'environnement chimique du fluorophore.

Selon la présente invention, les radicaux alkyle représentent des radicaux hydrocarbonés saturés, en chaîne droite ou ramifiés, de 1 à 30 atomes de carbone, de préférence de 1 à 20 atomes de carbone. On peut notamment citer, lorsqu'ils sont linéaires, les radicaux méthyle, éthyle, propyle, butyle, pentyle, hexyle, octyle, nonyle, décyle, dodécyle, hexadécyle et octadécyle.

On peut notamment citer, lorsqu'ils sont ramifiés ou substitués par un ou plusieurs radicaux alkyle, les radicaux isopropyle, tert-butyl, 2-éthylhexyle, 2-méthylbutyle, 2-méthylpentyle, 1-méthylpentyle et 3-méthylheptyle.

Le terme "halogénoalkyle" désigne un radical alkyle substitué par un ou plusieurs atomes d'halogène. Les radicaux halogénoalkyle incluent les radicaux perhalogénoalkyle et notamment les radicaux perfluoroalkyle de formule CnF₂ₙ₊₁.

Le terme "halogène" désigne un atome de chlore, brome, iode ou fluor.

Le terme « cycloalkyle » signifie un système de cycle non aromatique mono-ou multicyclique de 3 à 10 atomes de carbone, de préférence de 5 à 7 atomes de carbone. A titre d'exemple de cycloalkyle monocyclique, on peut citer notamment le cyclopentyle, le cyclohexyle, le cycloheptyle, et similaires. A titre d'exemple de groupe cycloalkyle multicyclique, on peut citer notamment la 1-décaline, le norbornyle, ou l'adamant-(1 ou 2-)yle.

Le terme « alcényle » désigne un groupe hydrocarboné aliphatique qui contient une double liaison carbone-carbone et qui peut être linéaire ou ramifié ayant de 2 à 6 atomes de carbone dans la chaîne. Ramifié signifie qu'un ou plusieurs groupes alkyle inférieurs, tels que le méthyle, l'éthyle ou le propyle, sont liés à une chaîne alcényle linéaire. Comme exemple de groupe alcényle, on peut citer notamment l'éthényle, le propényle, le *n*-butényle, l'*i*-butényle, le 3-méthylbut-2-ényle, ou le *n*-pentényle.

Le terme « alcynyle » désigne un groupe hydrocarboné aliphatique qui contient une triple liaison carbone-carbone et qui peut être linéaire ou ramifié ayant 2 à 6 atomes de carbone dans la chaîne, de préférence 2 à 4 atomes de carbone. Ramifié signifie qu'un ou plusieurs groupes alkyle inférieurs, tels que le méthyle, l'éthyle ou le propyle, sont liés à une chaîne alcynyle linéaire. Comme exemple de groupes alcynyles, on peut citer notamment l'éthynyle, le propynyle, le *n*-butynyle, le 2-butynyle, le 3-méthylbutynyle, et le *n*-pentynyle.

Le terme « aryle » désigne un système cyclique monocyclique ou multicyclique aromatique de 6 à 10 atomes de carbone. Comme exemple de groupes aryle, on peut citer notamment le phényle ou le naphtyle.

Le terme « aralkyle » désigne un groupe aryl-alkyl-, dans lequel l'aryle et l'alkyle sont tels que décrits dans le présent document. Comme exemple de groupes aralkyle, on peut citer notamment le benzyle, le 2-phénéthyle et le naphtlèneméthyle.

Le terme « groupe hétérocyclique » désigne un groupe carbocyclique substitué ou non substitué, mono- ou multicyclique dans lequel la partie cyclique comprend au moins un hétéroatome tel que O, N, ou S. L'azote et le soufre peuvent être éventuellement oxydés, et l'azote peut être éventuellement substitué dans les cycles aromatiques. Les groupes hétérocycliques comprennent les groupes hétéroaryles et les groupes hétérocycloalkyles.

Le terme « hétérocycloalkyle » désigne un groupe cycloalkyle dans lequel un ou plusieurs atomes de carbone de cycle sont substitués par au moins un atome choisi parmi O, N, ou S. A titre d'exemple de groupe hétérocycloalkyle, on peut citer notamment les groupes pyrrolidinyle, pyrrolinyle, imidazolidinyle imidazolinyle, pirazolidinyle, pirazolinyle, pyrazalinyle, pipéridyle, pipérazinyle, morpholinyle, thiomorpholinyle, tétrahydrofuranyle, dithiolyle, oxathiolyle, oxadiazolyle, oxathiazolyle, pyranyle, oxazinyle, oxathiazinyle, et oxadiazinyle..

Le terme « hétéroaryle » ou « hétéroaromatique » désigne un groupe contenant de 5 à 10 atomes de carbone dans lequel au moins un carbone de cycle est remplacer par au moins un atome choisi parmi -O-, -N-, or -S-. Comme exemple de groupe hétéroaryle, on peut citer notamment pyrrolyle, furanyle, thiényle, pirazolyle, imidazolyle, thiazolyle, isothiazolyle, isoxazolyle, oxazolyle, oxathiolyle, oxadiazolyle, triazolyle, oxatriazolyle, furazanyle, tétrazolyle, pyridyle, pyrazinyle, pyrimidinyle, pyridazinyle, triazinyle, indolyle, isoindolyle, indazolyle, benzofuranyle, isobenzofuranyle, purinyle, quinazolinyle, quinolyle, isoquinolyle, benzoimidazolyle, benzothiazolyle, benzothiophényle, thianaphthényle, benzoxazolyle, benzisoxazolyle, cinnolinyle, phthalazinyle, naphthyridinyle, and quinoxalinyle. Sont également compris dans la définition des groupes "hétéroaryle" les systèmes de cycles fusionnés incluant notamment les systèmes cycliques dans lequel le cycle aromatique est fusionné avec un cycle hétérocycloalkyle. Comme exemple de tels systèmes de cycles fusionnés, on peut citer notamment le phthalamide, l'anhydride phthalique, l'indoline, l'isoindoline, et la tetrahydroisoquinoline.

Le terme « hétéroarylalkyle » désigne un groupe aryl-hétéroaryl-, dans lequel l'hétéroaryle et l'alkyle sont tels que décrits dans le présent document.

Les termes « alkylène, cycloalkylène, hétérocycloalkylène, arylène, hétéroarylène » désignent respectivement des groupes bivalents alkyle, cycloalkyle, hétérocycloalkyle aryle et hétéroaryle, ces groupes étant tels que définis ci-dessus.

### FIGURES

Figure 1 : Température de transition solide liquide de composés bromure de 1-alkyl-3-[4-(5-phényl-oxazol-2-yl)-benzyl]-3H-imidazol-1-ium.
Figures 2 à 4 : Courbes de décroissance de la fluorescence obtenue avec les composés en C₁₆ des exemples 7 (figure 2), 8 (figure 3) et 9 (figure 4) par des photons CoC60 et des protons de 2MeV.
Figure 5 : Spectres d'émission et d'absorption du composé en C₁₆ de l'exemple 7.
Figure 6 : Dégradation de l'intensité de scintillation pour les composés des exemples 5 et 7, l'anthracène et le BC418 résultant des dégâts infligés aux matériaux par des protons de 2MeV.
Figures 7-8 : Pouvoir de discrimination neutron-gamma du composé de l'exemple 9 testé à partir d'une source radioactive de ²²Na+⁶⁰Co (rayonnement gamma) d'américium-béryllium (AmBe) (rayonnement de neutrons).

### EXEMPLES

Les exemples suivants illustrent l'invention, sans toutefois la limiter. Les produits de départ utilisés sont des produits connus ou préparés selon des modes opératoires connus.

Les pourcentages sont exprimés en poids, sauf mention contraire.

### Exemple 1

### Bromure de 1-Méthyle-3-[4-(5-phényl-oxazol-2-yl)-benzyl]-3H-imidazol-1-ium, 1a

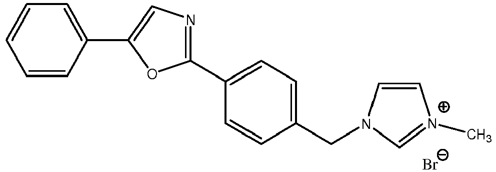

Dans un ballon préalablement purgé à l'argon anhydre, sont placés du 1-méthylimidazole (Aldrich) (0.257 mL, 0.265 g, 3.2 mmol), du 2-(4-bromométhylphényl)-5-phényl-oxazole [1] (1.013 g, 3.224 mmol) et du THF anhydre (15 mL).

Le mélange réactionnel est agité à 80°C pendant 12 heures. Durant la réaction, un précipité se forme.

Un solide blanc est isolé par filtration, lavé avec 2 fois 10 mL de THF et séché sous pression réduite à température ambiante. Le produit a été purifié par chromatographie flash (colonne de gel de silice, élution, DCM-MeOH, MeOH 1% à 7%) et caractérisé comme étant un sel d'imidazolium.
C₂₀H₁₈BrN₃O + 1.6 H₂O = 425,10 g.mol⁻¹
Rendement en solide incolore cristallin : (1.205 g, 94 %)

| | |
|---|---|
| C₂₀H₁₈BrN₃O; 1.6 H₂O | Calculé (%): C 56.57; H 4.49; N 10.03 |
| | Théorique (%): C 56,51 ; H 5.03; N 9.88 |

Pf = 190 ± 1 °C

Des expériences RMN ont été réalisées dans le CDCl₃. (¹H RMN,: 6 mg/0.3 mL CDCl₃)
¹H RMN (300 MHz, CDCl₃, 20°C): δ 10.72 ppm (s, 1 H, N-CH-N imidazolium), 8.11 (d, 2H, =CH- aromatique, ³J = 8.5 Hz), 7.67 (m, 4H, =CH- aromatique), 7.45 (m, 3H, =CH- aromatique), 7.32 (m, 3H, =CH- aromatique), 5.72 (s, 2H, Ph-CH₂-N), 4.09 (s, 3H, N-CH₃).
¹³C {¹H} RMN (75 MHz, CDCl₃, 20 °C): δ 36.83 ppm (Ph-CH₂-N), 52.78 (N-CH₃), 122.06 (C aromatique), 123.47 (C aromatique), 123.55 (C aromatique), 124.18 (C aromatique), 127.01 (C aromatique), 127.56 (C aromatique), 128.27 (C aromatique), 128.62 (C aromatique), 128,91 (C aromatique), 129,50 (C aromatique), 135.02 (C aromatique), 137.51 (N-CH-N imidazolium), 151.63 (C aromatique), 159.97 (C aromatique).
IR : 3141, 3088, 3057, 2998, 2859, 1766, 1739, 1649 (s), 1561 (s), 1493 (s), 1451 (s), 1420 (s), 1334, 1283, 1169 (s), 1133, 1065, 1021, 947, 872, 831, 764, 723, 689, 613 cm⁻¹.

### Exemple 2

### Bromure de 1-Hexyl-3-[4-(5-phényl-oxazol-2-yl)-benzyl]-3H-imidazol-1-ium, 1b

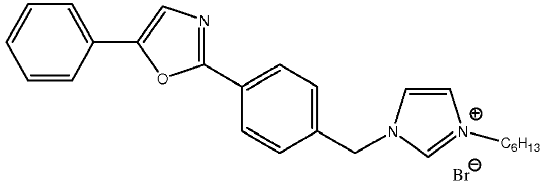

La procédure expérimentale est identique à celle du composé **1a** (1-hexylimidazole [2-5] 0.787 g, 5.2 mmol, 2-(4-bromométhyl-phényl)-5-phényloxazole 1.390 g, 4.4 mmol)
C₂₅H₂₈BrN₃O + 0.5 H₂O = 475.42 g.mol⁻¹
Rendement en solide incolore cristallin : (1.908 g, 92%)

| | |
|---|---|
| C₂₅H₂₈BrN₃O; 0.5 H₂O | Calculé (%): C 63.03; H 6.10; N 8.69 |
| | Théorique (%): C 63.16; H 6.15; N 8.84 |

Pf = 150 ± 1 °C
¹H RMN (300 MHz, CDCl₃, 20°C): δ 10.88 ppm (s, 1 H, N-CH-N imidazolium), 8.11 (d, 2H, =CH- aromatique, ³J = 8.2 Hz), 7.67 (m, 4H, =CH- aromatique), 7.40 (m, 5H, =CH- aromatique), 7.24 (m, 1H, =CH- aromatique), 5.77 (s, 2H, Ph-CH₂-N), 4.30 (t, 2H, N-CH₂-CH₂-(CH₂)₃-CH₃, ³J = 7.55 Hz), 1.92 (m, 2H, N-CH₂-CH₂-(CH₂)₃-CH₃), 1.31 (m, 6H, N-CH₂-CH₂-(CH₂)₃-CH₃), 0.87 (t, 3H, N-CH₂-CH₂-(CH₂)₃-CH₃, ³J = 6.9 Hz).
¹³C {¹H} RMN (75 MHz, CDCl₃, 20 °C): δ 13.79 (N-(CH₂)₅-CH₃) ppm, 22.24 (N-(CH₂)₄-CH₂-CH₃), 25.77 (N-(CH₂)₃-CH₂-CH₂-CH₃), 30.02 (N-(CH₂)₂-CH₂-(CH₂)₂-CH₃), 30.90 (N-CH₂-CH₂-(CH₂)₃-CH₃), 50.15 (N-CH₂-CH₂-(CH₂)₃-CH₃), 52.50 (Ph-CH₂-N), 122.25 (C aromatique), 122.41 (C aromatique), 123.44 (C aromatique), 124.09 (C aromatique), 126.91 (C aromatique), 127.52 (C aromatique), 128.16 (C aromatique), 128.54 (C aromatique), 128.86 (C aromatique), 129.50 (C aromatique), 135.26 (C aromatique), 136.70 (N-CH-N imidazolium), 151.53 (C aromatique), 159.96 (C aromatique).
IR : 3121, 3063, 2961, 2939, 2854, 1744, 1645, 1557 (s), 1491 (s), 1456 (s), 1418, 1358, 1324, 1154 (s), 1111, 1057, 1020, 949, 862, 838, 818, 764 (s), 716 (s), 687, 646, cm⁻¹.

### Exemple 3

### Bromure de 1-Octyl-3-[4-(5-phényl-oxazol-2-yl)-benzyl]-3H-imidazol-1-ium, 1c

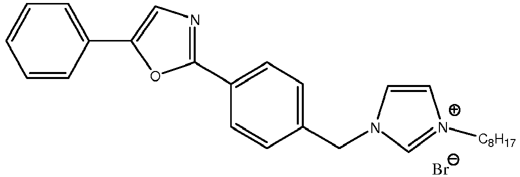

La procédure expérimentale est identique à celle du composé **1a** (1-octylimidazole [2-5] 0.865 g, 4.8 mmol, 2-(4-bromométhyl-phényl)-5-phényloxazole 1.495 g, 4.8 mmol)
C₂₇H₃₂BrN₃O + 0.25 H₂O = 498,97 g.mol⁻¹
Rendement en solide incolore cristallin : (2.134 g, 90 %)

| | |
|---|---|
| C₂₇H₃₂BrN₃O; 0.25 H₂O | Calculé (%): C 64.84; H 6.29; N 8.22 |
| | Théorique (%): C 64.99; H 6.57; N 8.42 |

Pf = 117 ± 1 °C
¹H RMN (300 MHz, CDCl₃, 20°C): δ 10.92 ppm (s, 1 H, N-CH-N imidazolium), 8.11 (d, 2H, =CH- aromatique, ³J = 8.2 Hz), 7.68 (m, 4H, =CH- aromatique), 7.45 (m, 3H, =CH- aromatique), 7.35 (m, 2H, =CH- aromatique), 7.23 (m, 1 H, =CH- aromatique), 5.77 (s, 2H, Ph-CH₂-N), 4.29 (t, 2H, N-CH₂-CH₂-(CH₂-)₅-CH₃, ³J = 7.6 Hz), 1.93 (m, 2H, N-CH₂-CH₂-(CH₂-)₅-CH₃), 1.24 (m, 10H, N-CH₂-CH₂-(CH₂-)₅-CH₃), 0.86 (t, 3H, N-CH₂-CH₂-(CH₂-)₅-CH₃, ³J = 6.6 Hz).
¹³C {¹H} RMN (75 MHz, CDCl₃, 20 °C): δ 13.94 (N-(CH₂)₇-CH₃) ppm, 22.45 (N-(CH₂)₆-CH₂-CH₃), 26.18 (N-(CH₂)₅-CH₂-CH₂-CH₃), 28.80 (N-(CH₂)₄-CH₂-(CH₂)₂-CH₃), 28.90 (N-(CH₂)₃-CH₂-(CH₂)₃-CH₃), 30.11 (N-(CH₂)₂-CH₂-(CH₂)₄-CH₃), 31.53 (N-CH₂-CH₂-(CH₂)₅-CH₃), 50.22 (N-CH₂-CH₂-(CH₂)₅-CH₃), 52.59(Ph-CH₂-N), 122.11 (C aromatique), 122.33 (C aromatique), 123.47 (C aromatique), 124.13 (C aromatique), 126.96 (C aromatique), 127.57 (C aromatique), 128.23 (C aromatique), 128.56 (C aromatique), 128.88 (C aromatique), 129.52 (C aromatique), 135.21 (C aromatique), 136.85 (N-CH-N imidazolium), 151.58 (C aromatique), 159.98 (C aromatique).
IR : 3065, 2926, 2854, 1648,1559 (s), 1489, 1454 (s), 1420, 1360, 1157 (s), 1117, 1020, 949, 860, 771, 719, 689 cm⁻¹.

### Exemple 4

### Bromure de 1-Décyl-3-[4-(5-phényl-oxazol-2-yl)-benzyl]-3H-imidazol-1-ium, 1d

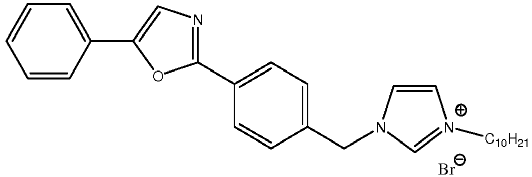

La procédure expérimentale est identique à celle du composé **1a** (1-décylimidazole [2-5] 0.973 g, 4.7 mmol, 2-(4 bromométhyl-phényl)-5-phényloxazole 1.383 g, 4.4 mmol)
C₂₉H₃₆BrN₃O + 0.5 H₂O = 531.53 g.mol⁻¹
Rendement en solide incolore cristallin : (2.111 g, 92 %)

| | |
|---|---|
| C₂₉H₃₆BrN₃O; 0.5 H₂O | Calculé (%): C 65.61; H 7.09; N 7.85 |
| | Théorique (%): C 65.53; H 7.02; N 7.91 |

Pf = 136 ± 1 °C
¹H RMN (300 MHz, CDCl₃, 20°C): δ 10.93 ppm (s, 1H, N-CH-N imidazolium), 8.12 (d, 2H, =CH- aromatique, ³J = 8.2 Hz), 7.68 (m, 4H, =CH- aromatique), 7.34 (m, 6H, =CH- aromatique), 5.77 (s, 2H, Ph-CH₂-N), 4.30 (t, 2H, N-CH₂-CH₂-(CH₂-)₇-CH₃, ³J = 7.5 Hz), 1.93 (m, 2H, N-CH₂-CH₂-(CH₂-)₇-CH₃), 1.24 (m, 14H, N-CH₂-CH₂-(CH₂-)₇-CH₃), 0.87(t, 3H, N-CH₂-CH₂-(CH₂-)₇-CH₃, ³J = 6.6 Hz).
¹³C {¹H} RMN (75 MHz, CDCl₃, 20 °C): δ 13.98 (N-(CH₂)₉-CH₃) ppm, 22.52 (N-(CH₂)₈-CH₂-CH₃), 26.17 (N-(CH₂)₇-CH₂-CH₂-CH₃), 28.84 (N-(CH₂)₆-CH₂-(CH₂)₂-CH₃), 29.10 (N-(CH₂)₅-CH₂-(CH₂)₃-CH₃), 29.24 (N-(CH₂)₄-CH₂-(CH₂)₄-CH₃), 29.30 (N-(CH₂)₃-CH₂-(CH₂)₅-CH₃), 30.11 (N-(CH₂)₂-CH₂-(CH₂)₆-CH₃), 31.70 (N-CH₂-CH₂-(CH₂)₇-CH₃), 50.18 (N-CH₂-CH₂-(CH₂)₇-CH₃), 52.53 (Ph-CH₂-N), 122.19 (C aromatique), 122.42 (C aromatique), 123.44 (C aromatique), 124.09 (C aromatique), 126.91 (C aromatique), 127.54 (C aromatique), 128.19 (C aromatique), 128.54 (C aromatique), 128.85 (C aromatique), 129.51 (C aromatique), 135.25 (C aromatique), 136.73 (N-CH-N imidazolium), 151.53 (C aromatique), 159.96 (C aromatique).
IR : 3078, 2992, 2922, 2851, 1749, 1645, 1558 (s), 1488, 1445, 1365, 1331, 1160 (s), 1118, 1059, 949, 876, 821, 767, 720, 691, 655 cm⁻¹.

### Exemple 5

### Bromure de 1-Dodécyl-3-[4-(5-phényl-oxazol-2-yl)-benzyl]-3H-imidazol-1-ium, 1e

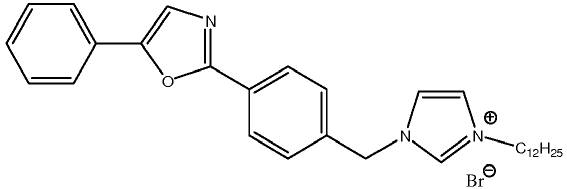

La procédure expérimentale est identique à celle du composé **1a** (1-dodécylimidazole [2-5] 1.262 g, 5.3 mmol, 2-(4-bromométhyl-phényl)-5-phényloxazole 1.342 g, 4.3 mmol)
C₃₁H₄₀BrN₃O + 0.15 H₂O = 553.28 g.mol⁻¹
Rendement en solide incolore cristallin : (2.23 g, 95 %)

| | |
|---|---|
| C₃₁H₄₀BrN₃O; 0.15 H₂O | Calculé (%) : C 67.25; H 7.28; N 7.40 |
| | Théorie (%) : C 67.30; H 7.34; N 7.59 |

Pf = 152 ± 1 °C
¹H RMN (300 MHz, CDCl₃, 20°C): δ 10.96 ppm (s, 1 H, N-CH-N imidazolium), 8.12 (d, 2H, =CH- aromatique, ³J = 8.5 Hz), 7.68 (m, 4H, =CH- aromatique), 7.45 (m, 3H, =CH- aromatique), 7.36 (m, 1H, =CH- aromatique), 7.30 (m, 1H, =CH- aromatique), 7.22 (m, 1H, =CH- aromatique), 5.77 (s, 2H, Ph-CH₂-N), 4.29 (t, 2H, N-CH₂- (CH₂)₁₀-CH₃, ³J = 7.5 Hz), 1.93 (m, 2H, N-CH₂-CH₂-(CH₂)₉-CH₃), 1.24 (m, 18H, N-CH₂-CH₂-(CH₂)₉-CH₃), 0.87 (t, 3H, N-CH₂- (CH₂)₁₀-CH₃, ³J = 6.72 Hz).
¹³C {¹H} RMN (75 MHz, CDCl₃, 20 °C): δ 14.03 (N-(CH₂)₁₁-CH₃) ppm, 22.58 (N-(CH₂)₁₀-CH₂-CH₃), 26.20 (N-(CH₂)₉-CH₂-CH₂-CH₃), 28.88 (N-(CH₂)₈-CH₂-(CH₂)₂-CH₃), 29.22 (N-(CH₂)₇-CH₂-(CH₂)₃-CH₃), 29.29 (N-(CH₂)₆-CH₂-(CH₂)₄-CH₃), 29.40 (N-(CH₂)₅-CH₂-(CH₂)₅-CH₃), 29.49 (N-(CH₂)₃-(CH₂)₂-(CH₂)₆-CH₃), 30.13 (N-(CH₂)₂-CH₂-(CH₂)₈-CH₃), 31.79 (N-CH₂-CH₂-(CH₂)₉-CH₃), 50.23 (N-CH₂-(CH₂)₁₀-CH₃), 52.61 (Ph-CH₂-N), 122.06 (C aromatique), 122.30 (C aromatique), 123.47 (C aromatique), 124.13 (C aromatique), 126.96 (C aromatique), 127.57 (C aromatique), 128.24 (C aromatique), 128.58 (C aromatique), 128.89 (C aromatique), 129.54 (C aromatique), 135.18 (C aromatique), 136.89 (N-CH-N imidazolium), 151.58 (C aromatique), 159.98 (C aromatique).
IR : 3082, 3015, 2919, 2848, 1735, 1560, 1489, 1443, 1364, 1159 (s), 1056, 949, 874, 812, 764, 719, 691, 654 cm⁻¹.

### Exemple 6 :

### Bromure de 3-[4-(5-Phényl-oxazol-2-yl)-benzyl]-1-tétradécyl-3H-imidazol-1-ium, 1f

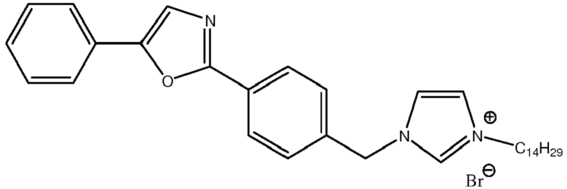

Dans un ballon préalablement purgé à l'argon anhydre, sont placés du 1-tétradécylimidazole [2-5] (1.023 g, 3.9 mmol), 2-(4-bromométhyl-phényl)-5-phényl-oxazole (1.215 g, 3.9 mmol) et du THF anhydre (15 mL). Le mélange réactionnel est agité à 80°C pendant 12 heures. (Le solvant est enlevé sous vide et le produit est lavé avec 3 fois 10 mL de Et₂O et séché sous pression réduite à température ambiante. Le produit a été purifié par chromatographie flash (colonne de gel de silice, élution, DCM-MeOH, MeOH 1% à 7%) et caractérisé comme étant un sel d'imidazolium.
C₃₃H₄₄BrN₃O = 578.63 g.mol⁻¹
Rendement en solide incolore cristallin : (2.092 g, 93 %)

| | |
|---|---|
| C₃₃H₄₄BrN₃O | Calculé (%): C 68.49; H 7.78; N 7.08 |
| | Théorique (%): C 68.50; H 7,66; N 7,20 |

Pf = 117 ± 1 °C
¹H RMN (300 MHz, CDCl₃, 20°C): δ 11.00_ppm (s, 1 H, N-CH-N imidazolium), 8.13 (d, 2H, =CH- aromatique, ³J = 8.2 Hz), 7.68 (m, 4H, =CH- aromatique), 7.46 (m, 3H, =CH- aromatique), 7.36 (m, 1 H, =CH- aromatique), 7.26 (m, 1 H, =CH- aromatique), 7.20 (m, 1 H, =CH- aromatique), 5.77 (s, 2H, Ph-CH₂-N), 4.30 (t, 2H, N-CH₂- (CH₂)₁₂-CH₃, ³J = 7.5 Hz), 1.94 (m, 2H, N-CH₂-CH₂-(CH₂)₁₁-CH₃), 1.24 (m, 22H, N-CH₂-CH₂-(CH₂)₁₁-CH₃), 0.87 (t, 3H, N-(CH₂)₁₃-CH₃, ³J = 6.7 Hz).
¹³C {¹H} RMN (75 MHz, CDCl₃, 20 °C): δ 14.03 (N-(CH₂)₁₃-CH₃) ppm, 22.58 (N-(CH₂)₁₂-CH₂-CH₃), 26.20 (N-(CH₂)₁₁-CH₂-CH₂-CH₃), 28.89 (N-(CH₂)₁₀-CH₂-(CH₂)₂-CH₃), 29.25 (N-(CH₂)₉-CH₂-(CH₂)₃-CH₃), 29.30 (N-(CH₂)₈-CH₂-(CH₂)₄-CH₃), 29.40 (N-(CH₂)₇-CH₂-(CH₂)₅-CH₃), 29.50 (N-(CH₂)₅-(CH₂)₂-(CH₂)₆-CH₃), 29.54 (N-(CH₂)₄-(CH₂)-(CH₂)₈-CH₃), 29.58 (N-(CH₂)₃-CH₂-(CH₂)₉-CH₃), 30.13 (N-(CH₂)₂-CH₂-(CH₂)₁₀-CH₃), 31.81 (N-CH₂-CH₂-(CH₂)₁₁-CH₃), 50.23 (N-CH₂-(CH₂)₁₂-CH₃), 52.61 (Ph-CH₂-N), 122.07 (C aromatique), 122.32 (C aromatique), 123.47 (C aromatique), 124.12 (C aromatique), 126.96 (C aromatique), 127.57 (C aromatique), 128.24 (C aromatique), 128.56 (C aromatique), 128.88 (C aromatique), 129.54 (C aromatique), 135.20 (C aromatique), 136.86 (N-CH-N imidazolium), 151.58 (C aromatique), 159.98 (C aromatique).
IR : 3127, 3096, 2917 (s), 2849 (s), 1609, 1589, 1556, 1470, 1440, 1363, 1330, 1186, 1160 (s), 1117, 1054, 951, 847, 818, 772, 717, 694, 661 cm⁻¹.

### Exemple 7 :

### Bromure de 1-Hexadécyl-3-[4-(5-phényl-oxazol-2-yl)-benzyl]-3H-imidazol-1-ium, 1g

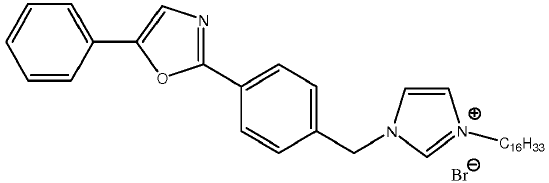

La procédure expérimentale est identique à celle du composé 1f (1-hexadécylimidazole [2-5] 1.385 g, 4.7 mmol, 2-(4-bromométhyl-phényl)-5-phényloxazole 1.489 g, 4.7 mmol)
C₃₅H₄₈BrN₃O = 606.68 g.mol⁻¹
Rendement en solide incolore cristallin : (2.680 g, 93%)

| | |
|---|---|
| C₃₅H₄₈BrN₃O | Calculé (%): C 68.86; H 8.39; N 7.17 |
| | Théorique (%): C 69.29; H 7.97; N 6.93 |

Pf = 97 ± 1 °C
¹H RMN (300 MHz, CDCl₃, 20°C): δ 10.93 ppm (s, 1 H, N-CH-N imidazolium), 8.12 (d, 2H, =CH- aromatique, ³J = 8.5 Hz), 7.68 (m, 4H, =CH- aromatique), 7.40 (m, 4H, =CH- aromatique), 7.25 (m, 2H, =CH- aromatique), 5.77 (s, 2H, Ph-CH₂-N), 4.30 (t, 3H, N-CH₂- (CH₂)₁₄-CH₃, ³J = 7.5 Hz), 1.93 (m, 2H, N-CH₂-CH₂-(CH₂)₁₃-CH₃), 1.23 (m, 26H, N-CH₂-CH₂-(CH₂)₁₃-CH₃), 0.88 (t, 3H, N-(CH₂)₁₅-CH₃, ³J = 6.7 Hz).
¹³C {¹H} RMN (75 MHz, CDCl₃, 20 °C): δ 14.06 (N-(CH₂)₁₅-CH₃) ppm, 22.63 (N-(CH₂)₁₄-CH₂-CH₃), 26.24 (N-(CH₂)₁₃-CH₂-CH₂-CH₃), 28.90 (N-(CH₂)₁₂-CH₂-(CH₂)₂-CH₃), 29.30 (N-(CH₂)₃-(CH₂)₉-(CH₂)₃-CH₃), 29.43 (N-(CH₂)₃-(CH₂)₉-(CH₂)₃-CH₃), 29.53 (N-(CH₂)₃-(CH₂)₉-(CH₂)₃-CH₃), 29.59 (N-(CH₂)₃-(CH₂)₉-(CH₂)₃-CH₃), 29.62 (N-(CH₂)₃-(CH₂)₉-(CH₂)₃-CH₃), 30.13 (N-(CH₂)₂-CH₂-(CH₂)₁₂-CH₃), 31.86 (N-CH₂-CH₂-(CH₂)₁₃-CH₃), 50.32 (N-CH₂-CH₂-(CH₂)₁₃-CH₃), 52.78 (Ph-CH₂-N), 121.75 (C aromatique), 121.95 (C aromatique), 123.52 (C aromatique), 124.19 (C aromatique), 127.06 (C aromatique), 127.62 (C aromatique), 128.39 (C aromatique), 128.62 (C aromatique), 128.93 (C aromatique), 129.57 (C aromatique), 134.99 (C aromatique), 137.31 (N-CH-N imidazolium), 151.66 (C aromatique), 160.00 (C aromatique).
IR : 3128, 3097, 2917 (s), 2850 (s), 1611, 1557, 1470, 1444, 1363, 1331, 1159 (s), 1122, 1058, 951, 846, 770, 720, 661 cm⁻¹.

### Exemple 8 :

### 1-Hexadécyl-3-[4-(5-phényl-oxazol-2-yl)-benzyl]-3H-imidazol-1-ium hexafluorophosphate, 2a

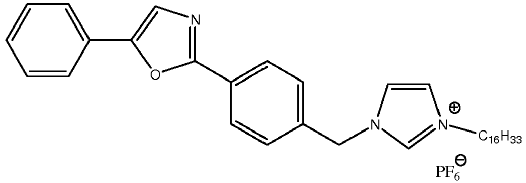

Dans un ballon, est introduit du bromure de 1-hexadécyl-3-[4-(5-phényloxazol-2-yl)-benzyl]-3H-imidazol-1-ium (1.008 g, 1.7 mmol) qui est dissout dans de l'éthanol (50 mL). On ajoute une solution aqueuse de hexafluorophosphate de potassium (0.490 g, 2.7 mmol, 10 mL). Le mélange est agité à température ambiante pendant 3 jours. Le précipité est filtré, lavé avec 3 fois 30 mL d'eau distillée et séché à pression réduite à température ambiante pendant 12 heures. Le produit est caractérisé par spectroscopie RMN.
C₃₅H₄₈F₆N₃OP = 671,74 g.mol⁻¹
Rendement en solide incolore cristallin : (0.994 g, 89%)
Pf = 114 ± 1 °C
¹H RMN (300 MHz, CDCl₃, 20°C): δ 8.80 ppm (s, 1 H, N-CH-N imidazolium), 8.12 (d, 2H, =CH- aromatique, ³J = 8.5 Hz), 7.70 (d, 2H, =CH- aromatique, ³J = 7.1 Hz), 7.47 (m, 5H, =CH- aromatique), 7.35 (m, 1H, =CH- aromatique), 7.21 (m, 2H, =CH- aromatique), 5.38 (s, 2H, Ph-CH₂-N), 4.18 (t, 3H, N-CH₂- (CH₂)₁₄-CH₃, ³J = 7.5 Hz), 1.89 (m, 2H, N-CH₂-CH₂-(CH₂)₁₃-CH₃), 1.23 (m, 26H, N-CH₂-CH₂-(CH₂)₁₃-CH₃), 0.88 (t, 3H, N-(CH₂)₁₅-CH₃, ³J = 6.7 Hz).
¹³C {¹H} RMN (75 MHz, CDCl₃, 20 °C): δ 14.05 (N-(CH₂)₁₅-CH₃) ppm, 22.62 (N-(CH₂)₁₄-CH₂-CH₃), 26.16 (N-(CH₂)₁₃-CH₂-CH₂-CH₃), 28.86 (N-(CH₂)₁₂-CH₂-(CH₂)₂-CH₃), 29.30 (N-(CH₂)₂-(CH₂)₁₀-(CH₂)₃-CH₃), 29.46 (N-(CH₂)₂-(CH₂)₁₀-(CH₂)₃-CH₃), 29.56 (N-(CH₂)₂ -(CH₂)₁₀-(CH₂)₃-CH₃), 29.64 (N-(CH₂)₂-(CH₂)₁₀-(CH₂)₃-CH₃), 29.75 (N-(CH₂)₂-(CH₂)₁₀-(CH₂)₃-CH₃), 31.86 (N-CH₂-CH₂-(CH₂)₁₃-CH₃), 50.31 (N-CH₂-CH₂-(CH₂)₁₄-CH₃), 52.99 (Ph-CH₂-N), 122.16 (C aromatique), 122.32 (C aromatique), 123.47 (C aromatique), 124.16 (C aromatique), 127.08 (C aromatique), 127.58 (C aromatique), 128.39 (C aromatique), 128.60 (C aromatique), 128.90 (C aromatique), 129.31 (C aromatique), 134.44 (C aromatique), 135.50 (N-CH-N imidazolium), 151.65 (C aromatique), 159.90 (C aromatique).
IR : 3165, 2922, 2851, 1561, 1468, 1417, 1384, 1163, 1116, 1057, 1023, 949, 840, 818, 770, 741, 720, 693, 654 cm⁻¹.

### Exemple 9 :

### 1-Hexadécyl-3-[4-(5-phényl-oxazol-2-yl)-benzyl]-3H-imidazol-1-ium bis(trifluorométhylsulfonyl)imide, 2b

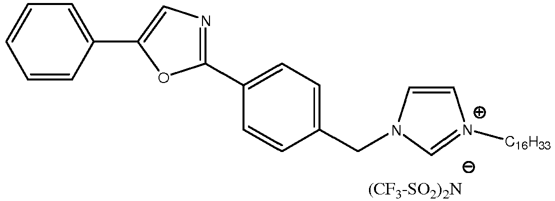

Dans un ballon, est introduit du bromure de 1-hexadécyl-3-[4-(5-phényl-oxazol-2-yl)-benzyl]-3H-imidazol-1-ium (1.007 g, 1.7 mmol) qui est ensuite dissout dans l'éthanol (55 mL). On ajoute du lithium bis(trifluorométhylsulfonyl)imide (0.731 g, 2.5 mmol). La solution est agitée à température ambiante pendant 3 jours. Le solvant est retiré sous pression réduite. Le solide est lavé avec 3 fois 30 mL d'eau distillée et séché sous pression réduite à température ambiante pendant 12 heures. Le produit est caractérisé par spectroscopie RMN.
C₃₇H₄₈F₆N₄O₅S₂ = 806.92 g.mol⁻¹
Rendement en solide incolore cristallin : (1.261 g, 94%)
Pf = 78 ± 1 °C
¹H RMN (300 MHz, CDCl₃, 20°C): δ 9.08 ppm (s, 1 H, N-CH-N imidazolium), 8.16 (d, 2H, =CH- aromatique, ³J = 8.2 Hz), 7.72 (d, 2H, =CH- aromatique, ³J = 7.1 Hz), 7.48 (m, 5H, =CH- aromatique), 7.37 (m, 1H, =CH- aromatique), 7.23 (d, 2H, =CH-aromatique, ³J = 7.1 Hz), 5.44 (s, 2H, Ph-CH₂-N), 4.22 (t, 2H, N-CH₂- (CH₂)₁₄-CH₃, ³J = 7.5 Hz), 1.89 (m, 2H, N-CH₂-CH₂-(CH₂)₁₃-CH₃), 1.24 (m, 26H, N-CH₂-CH₂-(CH₂)₁₃-CH₃), 0.88 (t, 3H, N-(CH₂)₁₅-CH₃, ³J = 6.7 Hz).
¹³C {¹H} RMN (75 MHz, CDCl₃, 20 °C): δ 14.05 (N-(CH₂)₁₅-CH₃) ppm, 22.63 (N-(CH₂)₁₄-CH₂-CH₃), 26.07 (N-(CH₂)₁₃-CH₂-CH₂-CH₃), 28.79 (N-(CH₂)₁₂-CH₂-(CH₂)₂-CH₃), 29.25 (N-(CH₂)₂-(CH₂)₁₀-(CH₂)₃-CH₃), 29.30 (N-(CH₂)₂-(CH₂)₁₀-(CH₂)₃-CH₃), 29.41 (N-(CH₂)₂ -(CH₂)₁₀-(CH₂)₃-CH₃), 29.53 (N-(CH₂)₂-(CH₂)₁₀-(CH₂)₃-CH₃), 29.59 (N-(CH₂)₂-(CH₂)₁₀-(CH₂)₃-CH₃), 29.63 (N-(CH₂)₂-(CH₂)₁₀-(CH₂)₃-CH₃), 29.97 (N-(CH₂)₂-(CH₂)₁₀-(CH₂)₃-CH₃), 31.86 (N-CH₂-CH₂-(CH₂)₁₃₋CH₃), 50.34 (N-CH₂-CH₂-(CH₂)₁₄-CH₃), 53.06 (Ph-CH₂-N) 119.80 (q, CF₃-, J = 321.1 Hz), 122.37 (C aromatique), 122.54 (C aromatique), 123.49 (C aromatique), 124.19 (C aromatique), 127.15 (C aromatique), 127.58 (C aromatique), 128.55 (C aromatique), 128.67 (C aromatique), 128.93 (C aromatique), 129.26 (C aromatique), 134.36 (C aromatique), 135.46 (N-CH-N imidazolium), 151.74 (C aromatique), 159.94 (C aromatique).
IR : 3141, 3084, 2919, 2851, 1937, 1849, 1800, 1590, 1560, 1493, 1466, 1416, 1351, 1181, 1138, 1059, 952, 906, 848, 824, 790, 764, 718, 687, 655, 615 cm⁻¹.

### Exemple 10 :

### 1-Hexadécyl-3-[4-(5-phényl-oxazol-2-yl)-benzyl]-3H-imidazol-1-ium dodécyl sulfate, 2c

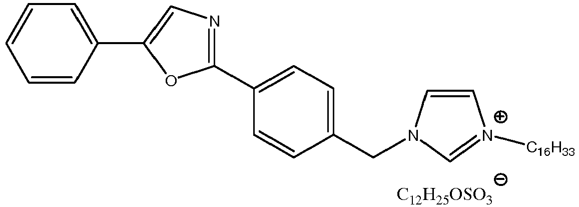

Dans un ballon, on introduit du bromure de 1-hexadécyl-3-[4-(5-phényl-oxazol-2-yl)-benzyl]-3H-imidazol-1-ium (1.005 g, 1.7 mmol) qui est ensuite dissout dans du méthanol (100 mL). On ajoute du sodium dodécyl sulfate (0.783 g, 2.7 mmol, 50 mL MeOH). Le mélange est agité à température ambiante pendant 12 heures.

Le solide blanc est isolé par filtration, lavé avec 3 fois 30 mL d'eau et séché sous pression réduite à température ambiante pendant 12 heures. Le produit est caractérisé par spectroscopie RMN.
C₄₇H₇₃N₃O₅S = 792.16 g.mol⁻¹
Rendement en solide incolore cristallin : (1.053 g, 80%)
Pf = 127 ± 1 °C
¹H RMN (300 MHz, CDCl₃, 20°C): δ 10.04 ppm (s, 1 H, N-CH-N imidazolium), 8.13 (d, 2H, =CH- aromatique, ³J = 8.2 Hz), 7.71 (d, 2H, =CH- aromatique, ³J = 7.1 Hz), 7.59 (d, 2H, =CH- aromatique, ³J = 8.2 Hz), 7.45 (m, 3H, =CH- aromatique), 7.36 (m, 1 H, =CH- aromatique), 7.19 (m, 2H, =CH- aromatique), 5.57 (s, 2H, Ph-CH₂-N), 4.25 (t, 2H, N-CH₂- (CH₂)₁₄-CH₃, ³J = 7.5 Hz),
4.10 (t, 2H, CH₃-(CH₂)₉-CH₂-CH₂-OSO₃⁻, ³J = 6.8 Hz), 1.90 (m, 2H, N-CH₂-CH₂-(CH₂)₁₃-CH₃), 1.68 (m, 2H, CH₃-(CH₂)₉-CH₂-CH₂-OSO₃⁻), 1.24 (m, 44H, N-CH₂-CH₂-(CH₂)₁₃-CH₃) et CH₃-(CH₂)₉-CH₂-CH₂-OSO₃⁻), 0.87 (m, 6H, N-(CH₂)₁₅-CH₃ et CH₃-(CH₂)₉-CH₂-CH₂-OSO₃⁻, ³J = 6.7 Hz).
¹³C {¹H} RMN (75 MHz, CDCl₃, 20 °C): δ 14.03 (N-(CH₂)₁₅-CH₃) et CH₃-(CH₂)₁₁-OSO₃⁻) ppm, 22.60 (N-(CH₂)₁₄-CH₂-CH₃) et CH₃-CH₂-(CH₂)₁₀-OSO₃⁻), 25.92 (N-(CH₂)₁₃-CH₂-CH₂-CH₃) et CH₃-CH₂-CH₂-(CH₂)₉-OSO₃⁻), 26.24 (N-(CH₂)₁₂-CH₂-(CH₂)₂-CH₃) et CH₃-(CH₂)₂-CH₂-(CH₂)₈-OSO₃⁻), 28.94 (N-(CH₂)₂-(CH₂)₁₀-(CH₂)₃-CH₃) et CH₃-(CH₂)₃-(CH₂)₆-(CH₂)₂-OSO₃⁻), 29.29 (N-(CH₂)₂-(CH₂)₁₀-(CH₂)₃-CH₃) et CH₃-(CH₂)₃-(CH₂)₆-(CH₂)₂-OSO₃⁻), 29.36 (N-(CH₂)₂-(CH₂)₁₀-(CH₂)₃-CH₃) et CH₃-(CH₂)₃-(CH₂)₆-(CH₂)₂-OSO₃⁻), 29.42 (N-(CH₂)₂-(CH₂)₁₀-(CH₂)₃-CH₃) et CH₃-(CH₂)₃-(CH₂)₆-(CH₂)₂-OSO₃⁻), 29.48 (N-(CH₂)₂-(CH₂)₁₀-(CH₂)₃-CH₃) et CH₃-(CH₂)₃-(CH₂)₆-(CH₂)₂-OSO₃⁻), 29.59 (N-(CH₂)₂-(CH₂)₁₀-(CH₂)₃-CH₃) et CH₃-(CH₂)₃-(CH₂)₆-(CH₂)₂-OSO₃⁻), 30.06 (CH₃-(CH₂)₉-CH₂-CH₂-OSO₃), 31.85 (N-CH₂-CH₂-(CH₂)₁₃-CH₃), 50.15 (CH₃-(CH₂)₉-CH₂-CH₂-OSO₃⁻), 52.74 (N-CH₂-CH₂-(CH₂)₁₄-CH₃), 67.73 (Ph-CH₂-N), 122.03 (C aromatique), 122.24 (C aromatique), 123.49 (C aromatique), 124.14 (C aromatique), 126.98 (C aromatique), 127.64 (C aromatique), 128.21 (C aromatique), 128.56 (C aromatique), 128.89 (C aromatique), 129.50 (C aromatique), 135.42 (C aromatique), 137.31 (N-CH-N imidazolium), 151.59 (C aromatique), 160.04 (C aromatique).
IR : 3123, 3071, 2919, 2850, 1634, 1552, 1468, 1381, 1248, 1154, 1097, 1061, 991, 869, 793, 768, 719, 693, 662, 620 cm⁻¹.

### Exemple 11 :

### 1-Hexadécyl-3-[4-(5-phényl-oxazol-2-yl)-benzyl]-3H-imidazol-1-ium hexadécyl sulfate, 2d

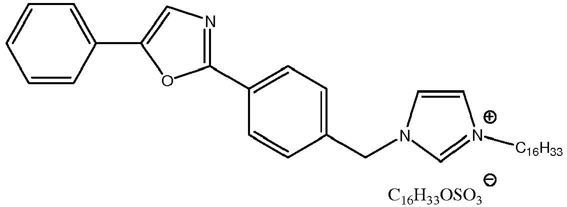

La procédure expérimentale est identique à celle du composé **2c** (bromure de 1-hexadécyl-3-[4-(5-phényl-oxazol-2-yl)-benzyl]-3H-imidazol-1-ium 1.010 g, 1.7 mmol, 60 mL EtOH), sodium hexadecyl sulfate (0.919 g, 2.7 mmol, 180 mL EtOH/60 mL H₂O).
C₅₁H₈₁N₃O₅S = 848.27 g.mol⁻¹
Rendement en solide incolore cristallin : (1.253 g, 89%)
Mp = 130 ± 1 °C
¹H RMN (300 MHz, CDCl₃, 20°C): δ 10.04 ppm (s, 1 H, N-CH-N imidazolium), 8.13 (d, 2H, =CH- aromatique, ³J = 8.2 Hz), 7.71 (d, 2H, =CH- aromatique, ³J = 7.4 Hz), 7.58 (d, 2H, =CH- aromatique, ³J = 7.9 Hz), 7.46 (m, 3H, =CH- aromatique), 7.36 (m, 1H, =CH- aromatique), 7.19 (d, 2H, =CH- aromatique, ³J = 11.0 Hz), 5.57 (s, 2H, Ph-CH₂-N), 4.25 (t, 2H, N-CH₂- (CH₂)₁₄-CH₃, ³J = 7.4 Hz), 4.10 (t, 2H, CH₃-(CH₂)₉-CH₂-CH₂-OSO₃⁻, ³J = 6.9 Hz), 1.90 (m, 2H, N-CH₂-CH₂-(CH₂)₁₃-CH₃), 1.68 (m, 2H, CH₃-(CH₂)₉-CH₂-CH₂-OSO₃⁻), 1.24 (m, 52H, N-CH₂-CH₂-(CH₂)₁₃-CH₃) et CH₃-(CH₂)₁₃-CH₂-CH₂-OSO₃⁻), 0.88 (m, 6H, N -CH₂-CH₂-(CH₂)₁₃-CH₃) et CH₃-(CH₂)₁₃-CH₂-CH₂-OSO₃⁻, ³J = 6.6 Hz)
¹³C {¹H} RMN (75 MHz, CDCl₃, 20 °C): δ 14.04 (N-(CH₂)₁₅-CH₃) et CH₃-(CH₂)₁₅-OSO₃⁻) ppm, 22.61 (N-(CH₂)₁₄-CH₂-CH₃) et CH₃-CH₂-(CH₂)₁₄-OSO₃⁻), 25.93 (N-(CH₂)₁₃-CH₂-CH₂-CH₃) et , CH₃-CH₂-CH₂-(CH₂)₁₃-OSO₃⁻), 26.24 (N-(CH₂)₁₂-CH₂-(CH₂)₂-CH₃) et CH₃-(CH₂)₂-CH₂-(CH₂)₁₂-OSO₃⁻), 28.94 (N-(CH₂)₂-(CH₂)₁₀-(CH₂)₃-CH₃) et CH₃-(CH₂)₃-(CH₂)₁₀-(CH₂)₂-OSO₃⁻), 29.29 (N-(CH₂)₂-(CH₂)₁₀-(CH₂)₃-CH₃) et CH₃-(CH₂)₃-(CH₂)₁₀-(CH₂)₂-OSO₃⁻), 29.37 (N-(CH₂)₂-(CH₂)₁₀-(CH₂)₃-CH₃) et CH₃-(CH₂)₃-(CH₂)₁₀-(CH₂)₂-OSO₃⁻), 29.43 (N-(CH₂)₂-(CH₂)₁₀-(CH₂)₃-CH₃) et CH₃-(CH₂)₃-(CH₂)₁₀-(CH₂)₂-OSO₃⁻), 29.48 (N-(CH₂)₂-(CH₂)₁₀-(CH₂)₃-CH₃) et CH₃-(CH₂)₃-(CH₂)₁₀-(CH₂)₂-OSO₃⁻), 29.64 (N-(CH₂)₂-(CH₂)₁₀-(CH₂)₃-CH₃) et CH₃-(CH₂)₃-(CH₂)₁₀-(CH₂)₂-OSO₃⁻), 30.06 (CH₃-(CH₂)₉-CH₂-CH₂-OSO₃⁻), 31.86 (N-CH₂-CH₂-(CH₂)₁₃-CH₃), 50.15 (CH₃-(CH₂)₉-CH₂-CH₂-OSO₃⁻), 52.74 (N-CH₂-CH₂-(CH₂)₁₄-CH₃), 67.73 (Ph-CH₂-N), 122.00 (C aromatique), 122.23 (C aromatique), 123.48 (C aromatique), 124.14 (C aromatique), 126.98 (C aromatique), 127.64 (C aromatique), 128.20 (C aromatique), 128.56 (C aromatique), 128.89 (C aromatique), 129.49 (C aromatique), 135.40 (C aromatique), 137.31 (N-CH-N imidazolium), 151.59 (C aromatique), 160.04 (C aromatique).
IR : 3123, 3071, 2918, 2850, 1630, 1552, 1470, 1409, 1381, 1248, 1153, 1113, 1063, 987, 872, 845, 793, 769, 719, 693, 662, 620 cm⁻¹.

### Exemple 12

### Stabilité thermique du bromure de 1-alkyl-3-[4-(5-phényl-oxazol-2-yl)-benzyl]-3H-imidazol-1-ium

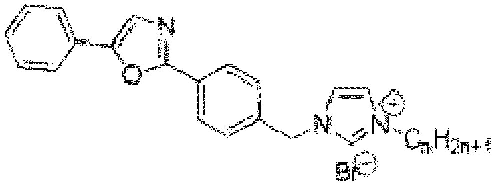

La stabilité thermique des composés bromure de 1-Alkyl-3-[4-(5-phényl-oxazol-2-yl)-benzyl]-3H-imidazol-1-ium a été étudiée en effectuant une analyse thermogravimétrique (ATG - DSC) au moyen d'un appareillage "SDT Q600" de la compagnie TA Instruments, muni d'un module DSC-TGA Standard. Les analyses ont été réalisées avec un balayage d'air de 50.0 ml/mn en capsule de platine et à la vitesse de 10°C/mn.

**Tableau 1**

| Bromure d'alkylimidazolium (n) | T _{dec} (°C) |
|---|---|
| n = 1 | 200-205 |
| n = 6 | 190-200 |
| n = 8 | 200-210 |
| n = 10 | 190-200 |
| n = 12 | 200-210 |
| n = 14 | 190-200 |
| n = 16 | 210-220 |

### Exemple 13

### Température de transition

Les radioluminophores bromure de 1-alkyl-3-[4-(5-phényl-oxazol-2-yl)-benzyl]-3H-imidazol-1-ium ont été caractérisés par une calorimétrie différentielle à balayage au moyen d'un appareillage "DSC Q1000" de la compagnie TA Instruments, muni d'une cellule DSC Standard et d'un cryostat RCS. Les analyses ont été réalisées avec un balayage d'azote de 50.0 ml/mn en capsule aluminium et à différentes vitesses.

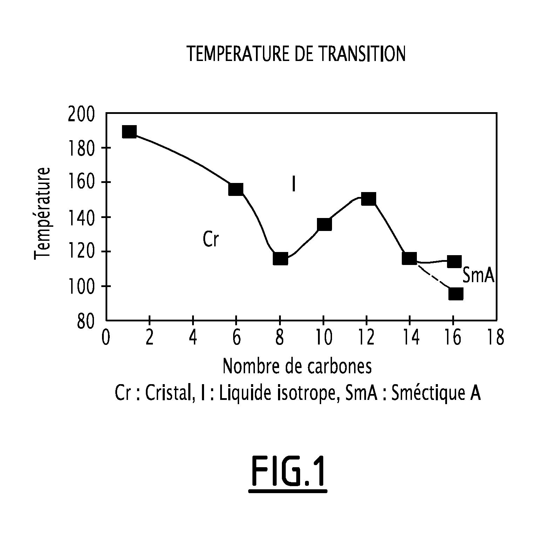

Les résultats obtenus sont représentés à la figure 1. Ils montrent le caractère modulable des propriétés physiques des composés de formule (I). En outre, de façon intéressante, une mésophase cristal liquide est observée pour la molécule présentant une chaîne carbonée en C₁₆. Les propriétés mésomorphes ont été caractérisées par des observations au microscope à lumière polarisée. L'émergence de la phase cristal-liquide en refroidissant le composé à partir de l'état liquide (isotrope) est caractéristique d'un Smectique A (formation de bâtonnets avec des unités positives). La calorimétrie différentielle indique une transition cristal-mésomorphe a 97°C (enthalpie 11,9 J/g) ainsi qu'une transition mésomorphe-liquide (point d'éclaircissement) a 115°C (2,0 J/g).

### Exemple 14

### Pouvoir de détection

La discrimination n-γ repose sur la comparaison, à composantes rapides égales, des composantes lentes résultant des processus d'ionisation induits respectivement par les rayonnements gamma et les protons de recul produits par les neutrons [6-11]. Le moyen le plus fin d'effectuer la mesure d'un déclin de fluorescence est d'exciter impulsionnellement (impulsion nanoseconde) l'échantillon et de reconstituer statistiquement sa réponse par une technique de coïncidence, ceci en utilisant un photomultiplicateur fonctionnant en régime de photoélectron unique.

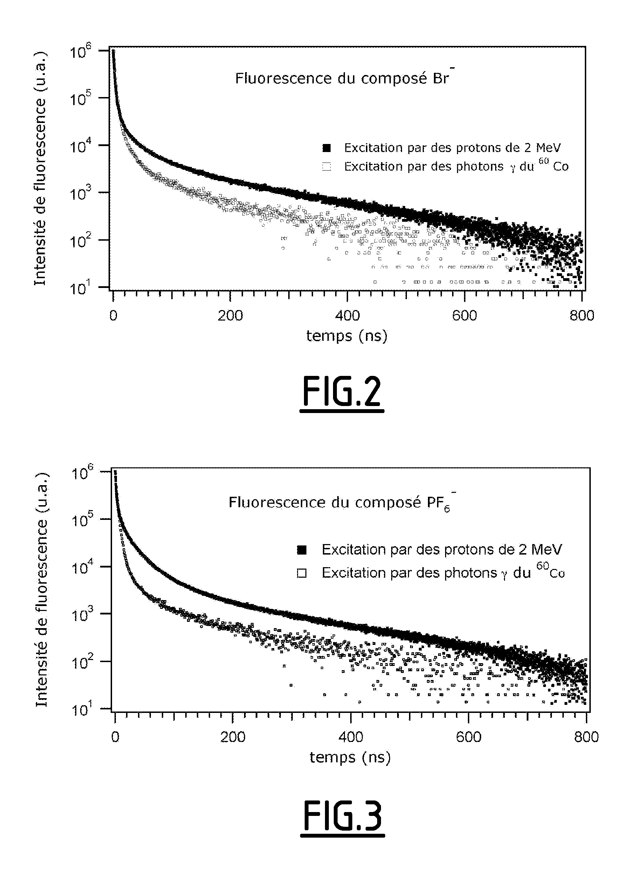

Les premières mesures de déclins excités par des protons pulsés nanoseconde d'énergies comprises entre 1 et 4 MeV (accélérateur électrostatique 4MV) ont été réalisées dans les conditions de production d'un proton de recul pour induire une réponse du détecteur. A titre d'exemple, les figures 2 à 4 représentent des courbes de décroissance de la fluorescence obtenues avec les composés des exemples 7 (fig. 1), 8 (fig. 2) et 9 (fig. 3)(chaînes alkyles C₁₆) excité par des photons du Co60 et des protons de 2 MeV. On observe bien la présence de deux composantes, rapide (t<20 ns ; t : temps)) et lente (t≥20 ns). La composante rapide est de type exponentiel. La durée de vie de l'état excité fluorescent du composé est comprise entre une et deux nanosecondes. Comme prédit par la théorie, la loi de décroissance de la composante lente est de type t ³/₂, ce qui indique une diffusion isotrope des porteurs des charges avant leur recombinaison mutuelle. Le comportement, en matière de détection, de ces nouvelles molécules est donc tout à fait semblable à celui des anciens produits et peut donc les remplacer sans nécessiter de modification importante des systèmes de détection (photomultiplicateur) et d'acquisition de données (électronique et informatique de traitement).

### Exemple 15

### Spectre d'absorption et d'émission

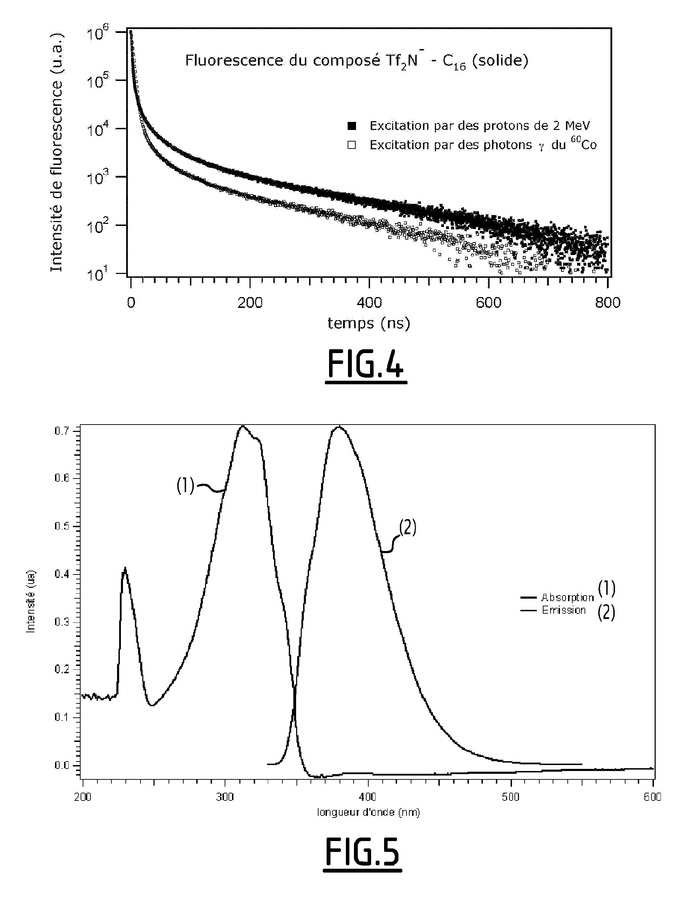

On a également observé que l'émission de fluorescence du composé en C₁₆ de l'exemple 7 se situait bien dans le domaine spectral de l'ultraviolet, vers 400 nm (voir spectres d'émission et d'absorption en figure 5), ce qui est tout à fait compatible avec un remplacement des scintillateurs actuels sans modification des chaînes de détection et d'analyse.

### Exemple 16

### Tenue aux rayonnements

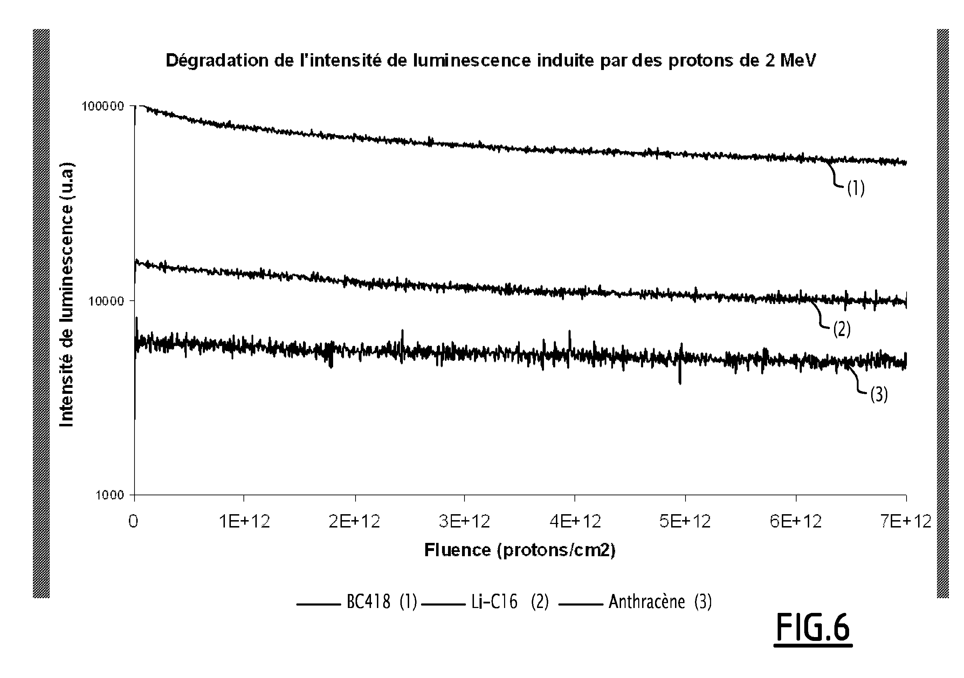

La diminution de l'intensité de la fluorescence résultant des dégâts infligés aux matériaux par des protons de 2 MeV a également été étudiée. On observe un très bon comportement des molécules selon l'invention (voir la figure 6) si on compare leurs tenues à celles de matériaux concurrents, tel que le BC418 commercialisé par la société Bicron, filiale de Saint-Gobain. Ainsi, pour une intensité de fluorescence donnée à fluence nulle, on remarque que la perte d'intensité du composé en C₁₆ de l'exemple 7 avec la fluence n'est pas très différente de celle du BC418.

### Exemple 17

### Pouvoir de discrimination n-γ

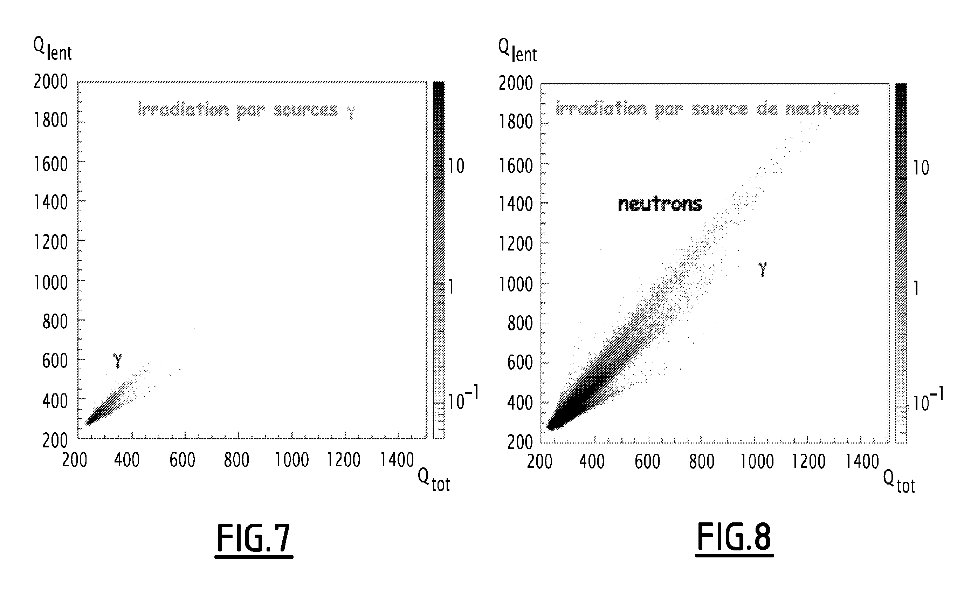

Le pouvoir de discrimination neutron gamma du composé de l'exemple 9 a été testé en situation réelle à partir d'une source radioactive de sodium et de cobalt 60 (²²Na + ⁶⁰CO) d'américium-béryllium (AmBe). L'analyse des composantes rapides et lentes des déclins de fluorescence permet de tracer le graphe représentant, en abscisse, l'intégrale du signal (Q tot) et, en ordonnée, l'intégrale de la composante lente (Q lent). On observe ainsi une différence très marquée entre l'intensité de la composante lente obtenue avec le rayonnement gamma (fig. 7) et celle obtenue avec les neutrons (fig. 8). Ceci met en évidence, de manière indiscutable, la propriété de discrimination neutron gamma des composés de formule (I).

### Exemple 18

### Polymère méthacrylate de méthyle contenant 52% (en poids) de sel d'hexafluorophosphate.

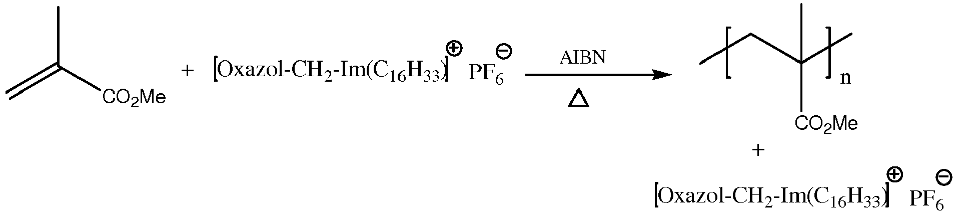

Dans un récipient en verre, sont introduits de l'hexafluorophosphate de 1-hexadécyl-3-[4-(5-phényl-oxazol-2-yl)-benzyl]-3H-imidazol-1-ium (5,10 g, 8,4 mmol) et du méthacrylate de méthyle (MMA, Prolabo) (10,45 mL, 9,80 g, 98,0 mmol). Le mélange est chauffé entre 40° et 60°C jusqu'à dissolution complète du sel d'hexafluorophosphate. Du 2'-Azobis(2-méthylpropionitrile) (AIBN, Acros, 12 mg) est ajouté. Le mélange est chauffé à différentes températures (Tᵢ: °C) pendant différents intervalles de temps (tᵢ : heures).
1/ (T₁, t₁) : (60, 3)
2/ (T₂, t₂) : (96, 1)
3/ (T₃, t₃) : (108, 2)

A la fin, pour terminer le processus de polymerisation, le mélange est chauffé à 115°C pendant 10-30 mn. Le mélange est ensuite refroidi dans un bain de glace (0°C) pendant 20 mn. Le polymère de méthacrylate de méthyle incorporant le sel d'hexafluorophosphate (52% en poids) est un solide transparent.

| Expérience n° | Wt %, (salt: g/MMA: g) | AIBN (mg) | T₁, T₂, T₃ (°C) | t₁, t₂, t₃ (h) |
|---|---|---|---|---|
| EB083 | 0 (0/2.817) | 8 | 60, 96, 108 | 2.5, 3, 0 |
| EB095 | 11 (0.10/0.939) | 4 | 60, 96, 115 | 3, 0, 3 |
| EB096 | 21 (0.20/0.939) | 4 | 60, 96, 108 | 3, 3.5, 0 |
| EB097 | 43 (0.20/0.469) | 2 | 60, 96, 108 | 3, 4, 0 |
| EB099 | 43 (0.20/0.469) | 2 | 60, 96, 115 | 3, 2.5, 1 |
| EB100 | 43 (0.20/0.469) | 2 | 60, 96, 108 | 3, 2, 2 |
| EB101 | 53 (0.25/0.469) | 2 | 60, 96, 108 | 3, 1.5, 2 |
| EB102 | 64 (0.30/0.469) | 3 | 60, 96, 108 | 3, 1, 2 |
| EB103 | 75 (0.35/0.469) | 3 | 60, 96, 108 | 3, 1, 2.5 |

A noter que le méthacrylate de méthyle a été préalablement séché sur CaH₂, purifié par distillation sous vide et stocké à -25°C.

AIBN a été purifié par recristallisation à partir du méthanol à 0°C.

### REFERENCES

[1] H.-J. Meyer and T. Wolff, Chem. Euro. J., 2000, 15, 2809-2817: Water-binding Solid Scintillators: Synthesis, Emission properties and test in 3H and 14C counting*,*
[2] S. Khabnadideh, Z. Rezaei, A. Khalafi-Nezhad, R. Bahrinajafi, R. Mohamadi, A. A. Farrokhroz, Bioorganic & Medicinal Chemistry Letters 13 (2003) 2863-2865 - Synthesis of N-alkyated Derivatives of Imidazole as Antibacterial Agents
[3] Jen-Yen Cheng and Yen-Ho Chu*, Tetrahedron Letters 47 (2006) 1575-1579,
[4] Martin Tosoni, Sabine Laschat*, and Angelika Baro., Helvetica Chimica Acta Vol. 87 (2004)
[5] Wang, Xixin; Zhao, Jianling; Yang, Hao; Chen, Wentao; Zhu, Zhijia. Department of Chemistry, Nanyang Teacher's College, Nanyang, Peop. Rep. China. Huaxue Shiji (2001), 23(5), 306-307. Publisher: Huagongbu Huaxue Shiji Xinsizhan, CODEN: HUSHDR ISSN: 0258-3283. Journal written in Chinese. CAN 136:279384 AN 2001:877665 CAPLUS
[6] J.-M. Jung, Thèse de Doctorat de l'Université Louis Pasteur n°998, Strasbourg, 1991
[7] D. Paligoric, J. Klein, Int. J. Rad. Phys. Chem. 4 (1972) 359
[8] J. Klein, R. Voltz, Phys. Rev. Letters 36 (1976) 1214
[9] J. Klein, R. Voltz, Can. J. Chem. 55 (1977) 2102
[10] J. Klein, J. Chim. Phys. 80 (1983) 627
[11] G. Klein, R. Voltz, M. Schott, Chem. Phys. Lett. 16 (1972) 340 ; id. 19 (1973) 391

## Revendications

1. Composé fluorophore répondant à la formule (la) : 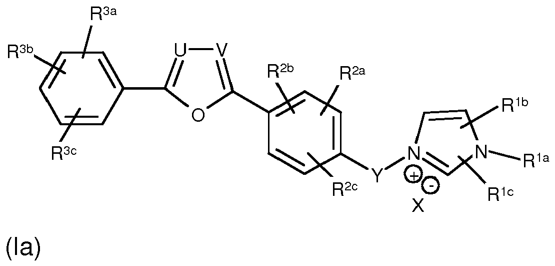 dans laquelle :
R^{1a} est un groupe alkyle en C₁-C₃₀ ;
R^{1b} et R^{1c} sont H ; 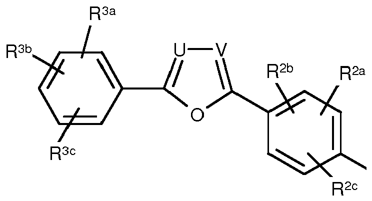 est un groupe fluorophore dans lequel :
R^{2a}, R^{2b}, R^{2c}, R^{3a}, R^{3b}, R^{3c} sont, à chaque occurrence, chacun indépendamment choisis parmi H, les groupes alkyles en C₁-C₃₀, et cycloalkyles en C₃-C₇;
Y est un groupe alkylène en C₁-C₆
U, V représentent indépendamment CR²⁶ ou N, étant entendu que l'un au moins de U ou V représente N ;
R²⁶ est H, C₁-C₆ alkyle, OR²², NR²³R²⁴, NO₂, C(=O)R²², CO₂R²², OC(=O)R²², C(=O)NR²³R²⁴, OC(=O)NR²³R²⁴, SO₃H, PO₄H, CN ;
R²² est, à chaque occurrence, indépendamment choisi parmi H, alkyle en C₁-C₆,
aryle en C₆-C₁₀ et aralkyle ;
R²³ et R²⁴ sont, à chaque occurrence, indépendamment choisis parmi H, alkyle en C₁-C₆ et aryle en C₆-C₁₀, ou bien R²³ et R²⁴ forment avec l'atome d'azote auquel ils sont attachés un groupe hétérocyclique de 3 à 7 chaînons ;
X- est un anion organique ou inorganique.

2. Composé selon la revendication 1, dans lequel R^{1a} est un groupe alkyle en C₁-C₁₆.

3. Composé selon l'une quelconque des revendications 1 à 2, dans lequel U est CR²⁶.

4. Composé selon l'une quelconque des revendications 1 à 2, dans lequel U est CH.

5. Composé selon l'une quelconque des revendications 1 à 4, dans lequel Y est un groupe CH₂.

6. Composé selon l'une quelconque des revendications 1 à 5, dans lequel X-est un anion choisi parmi un halogénure, P(R⁴)₆⁻, B(R⁴)₄⁻, SCN⁻, (R⁵SO₂)₂N⁻, R⁵OSO₃, R⁵SO₃⁻, carborane, carbonate (CO₃²⁻), hydrogénocarbonate (HCO₃⁻), alcoolate (R⁴O⁻), carboxylate (R⁴COO⁻), amidure (NH₂⁻), phosphate (PO₄⁻), SiF₆⁻, SbF₆⁻, I₃⁻, nitrate (NO₃⁻), oxyde d'halogénure, silicate, sulfate (SO₄⁻), sulfonate (R⁴SO₃⁻), cyanure (CN⁻), carbanions, ou métallate ; où :
R⁴ est, à chaque occurrence, un groupe indépendamment choisi parmi un atome d'halogène, un groupe alkyle en C₁-C₆, un groupe aryle en C₆-C₁₀, un groupe aralkyle ;
R⁵ est, à chaque occurrence, un groupe indépendamment choisi parmi alkyle en C₁-C₂₀, halogénoalkyle en C₁-C₂₀, aryle en C₆-C₁₀, aralkyle.

7. Composé selon l'une quelconque des revendications 1 à 6, dans lequel X-est choisi parmi Br⁻, PF₆⁻, BF₄⁻, (CF₃SO₂)₂N⁻, C₁₂H₂₅OSO₃⁻, C₁₆H₃₃OSO₃-, CF₃SO₃⁻.

8. Composé selon la revendication 1, dans lequel le composé de formule (la) est choisi parmi :
- bromure de 1-méthyle-3-[4-(5-phényl-oxazol-2-yl)-benzyl]-3H-imidazol-1-ium
- bromure de 1-hexyl-3-[4-(5-phényl-oxazol-2-yl)-benzyl]-3H-imidazol-1-ium
- bromure de 1-octyl-3-[4-(5-phényl-oxazol-2-yl)-benzyl]-3H-imidazol-1-ium
- bromure de 1-décyl-3-[4-(5-phényl-oxazol-2-yl)-benzyl]-3H-imidazol-1-ium
- bromure de 1-dodécyl-3-[4-(5-phényl-oxazol-2-yl)-benzyl]-3H-imidazol-1-ium
- bromure de 3-[4-(5-phényl-oxazol-2-yl)-benzyl]-1-tétradécyl-3H-imidazol-1-ium
- bromure de 1-hexadécyl-3-[4-(5-phényl-oxazol-2-yl)-benzyl]-3H-imidazol-1-ium
- hexafluorophosphate de 1-hexadécyl-3-[4-(5-phényl-oxazol-2-yl)-benzyl]-3H-imidazol-1-ium
- 1-hexadécyl-3-[4-(5-phényl-oxazol-2-yl)-benzyl]-3H-imidazol-1-ium bis(trifluorométhylsulfonyl)imide
- 1-hexadécyl-3-[4-(5-phényl-oxazol-2-yl)-benzyl]-3H-imidazol-1-ium dodécyl sulfate
- 1-hexadécyl-3-[4-(5-phényl-oxazol-2-yl)-benzyl]-3H-imidazol-1-ium hexadécyl sulfate.

9. Utilisation pour la détection des rayonnements gamma, X, neutron, proton, d'un composé fluorophore tel que défini selon l'une quelconque des revendications 1 à 8.

10. Utilisation d'un composé tel que défini selon l'une quelconque des revendications 1 à 8, pour la fabrication d'un instrument de radiodétection, de dosimétrie industrielle ou médicale.

11. Utilisation d'un composé fluorophore tel que défini selon l'une quelconque des revendications 1 à 8, pour la discrimination des rayonnements proton/gamma, proton/X, neutron/gamma, neutron/X, alpha/gamma, alpha/X.

12. Matériau comprenant un composé selon l'une quelconque des revendications 1 à 8.

13. Matériau selon la revendication 12, **caractérisé en ce que** le matériau est un matériau plastique transparent.

14. Procédé de préparation d'un composé de formule (la) tel que défini selon l'une quelconque des revendications 1 à 8, ledit procédé comprenant la réaction d'un composé de formule (II) avec un composé de formule (III) : 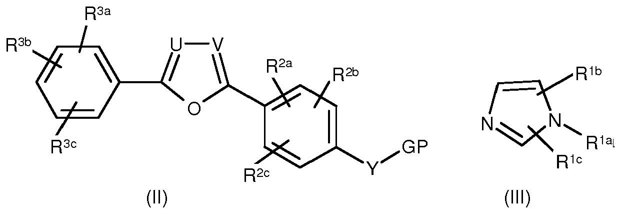 dans lesquelles R^{1a}, R^{1b}, R^{1c}, R^{2a}, R^{2b}, R^{2c}, R^{3a}, R^{3b}, R^{3c}, U, et Y sont tels que définis selon l'une quelconque des revendications 1 à 8, et GP représente un groupe partant.

15. Procédé selon la revendication 14, **caractérisé en ce que** la réaction est réalisée dans un solvant polaire aprotique.

## Patentansprüche

1. Fluorophorverbindung gemäß der Formel (la): 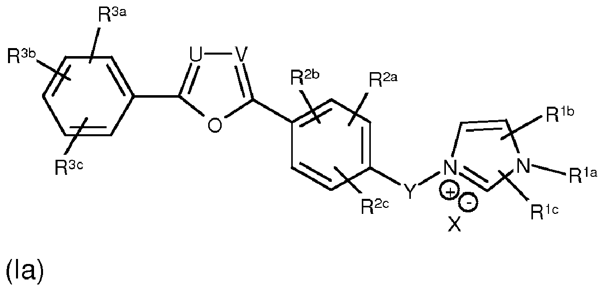 wobei:
R^{1a} eine C₁-C₃₀ Alkylgruppe ist;
R^{1b} und R^{1c} H sind; 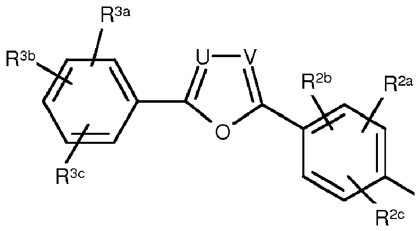 eine Fluorophorgruppe ist, wobei:
R^{2a}, R^{2b}, R^{2c}, R^{3a}, R^{3b}, R^{3c} bei jedem Vorkommen, jeweils unabhängig voneinander ausgewählt werden aus H, C₁-C₃₀ Alkyl- und C₃-C₇ Cycloalkylgruppen;
Y eine C₁-C₆ Alkylengruppe ist;
U, V unabhängig voneinander CR²⁶ oder N darstellen, mit der Maßgabe, dass mindestens eine aus U oder V N darstellt;
R²⁶ H, C₁-C₆ Alkyl, OR²², NR²³R²⁴, NO₂, C(=O)R²², CO₂R²², OC(=O)R²², C(=O)NR²³R²⁴, OC(=O)NR²³R²⁴, SO₃H, PO₄H, CN ist;
R²² bei jedem Vorkommen, jeweils unabhängig voneinander ausgewählt wird aus H, C₁-C₆ Alkyl, C₆-C₁₀ Aryl und Aralkyl;
R²³ und R²⁴ bei jedem Vorkommen, jeweils unabhängig voneinander ausgewählt werden aus H, C₁-C₆ Alkyl und C₆-C₁₀ Aryl oder R²³ und R²⁴ bilden mit dem Stickstoffatom, an das sie gebunden sind, eine heterocyclische Gruppe mit 3 bis 7 Gliedern;
X⁻ ein organisches oder anorganisches Anion ist.

2. Verbindung gemäß Anspruch 1, wobei R^{1a} eine C₁-C₁₆ Alkylgruppe ist.

3. Verbindung gemäß einem der Ansprüche 1 bis 2, wobei U CR²⁶ ist.

4. Verbindung gemäß einem der Ansprüche 1 bis 2, wobei U CH ist.

5. Verbindung gemäß einem der Ansprüche 1 bis 4, wobei Y eine CH₂ Gruppe ist.

6. Verbindung gemäß einem der Ansprüche 1 bis 5, wobei X⁻ ein Anion ist, welches ausgewählt wird aus einem Halogen, P(R⁴)₆⁻, B(R⁴)₄⁻, SCN⁻, (R⁵SO₂)₂N⁻, R⁵OSO₃, R⁵SO₃⁻, Carboran, Carbonat (CO₃²⁻), Hydrogencarbonat (HCO₃⁻), Alkoholat (R⁴O⁻), Carboxylat (R⁴COO⁻), Amid (NH₂⁻), Phosphat (PO₄⁻), SiF₆⁻, SbF₆⁻, I₃⁻, Nitrat (NO₃⁻), Halogenoxid, Silicat, Sulfat (SO₄⁻), Sulfonat (R⁴SO₃⁻), Cyanid (CN⁻), Carbanion oder Metallat; wobei:
R⁴ bei jedem Vorkommen eine Gruppe ist, welche jeweils unabhängig ausgewählt wird aus einem Halogenatom, einer C₁-C₆ Alkylgruppe, einer C₆-C₁₀ Arylgruppe, einer Aralkylgruppe;
R⁵ bei jedem Vorkommen eine Gruppe ist, welche jeweils unabhängig ausgewählt wird aus C₁-C₂₀ Alkyl, C₁-C₂₀ Halogenalkyl, C₆-C₁₀ Aryl, Aralkyl.

7. Verbindung gemäß einem der Ansprüche 1 bis 6, wobei X⁻ ausgewählt wird aus Br⁻, PF₆⁻, BF₄⁻, (CF₃SO₂)₂N⁻, C₁₂H₂₅OSO₃⁻, C₁₆H₃₃OSO₃⁻, CF₃SO₃⁻.

8. Verbindung gemäß dem Anspruch 1, wobei die Verbindung nach Formel (la) ausgewählt wird aus:
- 1-Methyl-3-[4-(5-phenyl-oxazol-2-yl)-benzyl]-3H-imidazol-1-ium bromid
- 1-Hexyl-3-[4-(5-phenyl-oxazol-2-yl)-benzyl]-3H-imidazol-1-ium bromid
- 1-Octyl-3-[4-(5-phenyl-oxazol-2-yl)-benzyl]-3H-imidazol-1-ium bromid
- 1-Decyl-3-[4-(5-phenyl-oxazol-2-yl)-benzyl]-3H-imidazol-1-ium bromid
- 1-Dodecyl-3-[4-(5-phenyl-oxazol-2-yl)-benzyl]-3H-imidazol-1-ium bromid
- 3-[4-(5-phenyl-oxazol-2-yl)-benzyl]-1-Tetradecyl-3H-imidazol-1-ium bromid
- 1-Hexadecyl-3-[4-(5-phenyl-oxazol-2-yl)-benzyl]-3H-imidazol-1-ium bromid
- 1-Hexadecyl-3-[4-(5-phenyl-oxazol-2-yl)-benzyl]-3H-imidazol-1-ium hexafluorphosphat
- 1-Hexadecyl-3-[4-(5-phenyl-oxazol-2-yl)-benzyl]-3H-imidazol-1-ium bis(trifluormethylsulfonyl)imid
- 1-Hexadecyl-3-[4-(5-phenyl-oxazol-2-yl)-benzyl]-3H-imidazol-1-ium dodecylsulfat
- 1-Hexadecyl-3-[4-(5-phenyl-oxazol-2-yl)-benzyl]-3H-imidazol-1-ium hexadecylsulfat.

9. Verwendung einer Fluorophorverbindung, wie in einem der Ansprüche 1 bis 8 definiert, zur Detektion von Gammastrahlung, Röntgenstrahlung, Neutronenstrahlung.

10. Verwendung einer Fluorophorverbindung, wie in einem der Ansprüche 1 bis 8 definiert, zur Herstellung eines Radargeräts, eines industriellen Dosimetrieinstruments oder eines medizinischen Dosimetrieinstruments.

11. Verwendung einer Fluorophorverbindung, wie in einem der Ansprüche 1 bis 8 definiert, zur Unterscheidung von Protonen-/ Gammastrahlung, Protonen-/ Röntgenstrahlung, Neutronen-/ Gammastrahlung, Neutronen-/ Röntgenstrahlung, Alpha-/ Gammastrahlung, Alpha-/ Röntgenstrahlung.

12. Material, umfassend eine Verbindung gemäß einem der Ansprüche 1 bis 8.

13. Material gemäß dem Anspruch 12, **dadurch gekennzeichnet, dass** das Material ein durchsichtiges Kunststoffmaterial ist.

14. Verfahren zur Herstellung einer Verbindung der Formel (la), wie in einem der Ansprüche 1 bis 8 definiert, wobei das Verfahren die Reaktion einer Verbindung der Formel (II) mit einer Verbindung der Formel (III) umfasst: 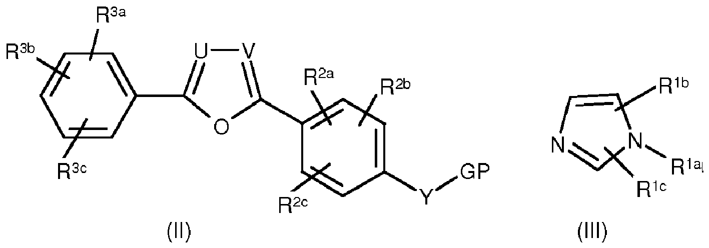 wobei R^{1a}, R^{1b}, R^{1c}, R^{2a}, R^{2b}, R^{2c}, R^{3a}, R^{3b}, R^{3c}, U und Y wie in einem der Ansprüche 1 bis 8 definiert sind und GP eine Abgangsgruppe darstellt.

15. Verfahren gemäß dem Anspruch 14, **dadurch gekennzeichnet, dass** die Reaktion in einem polaren, aprotischen Lösungsmittel durchgeführt wird.

## Claims

1. A fluorophore compound fitting the formula (Ia): 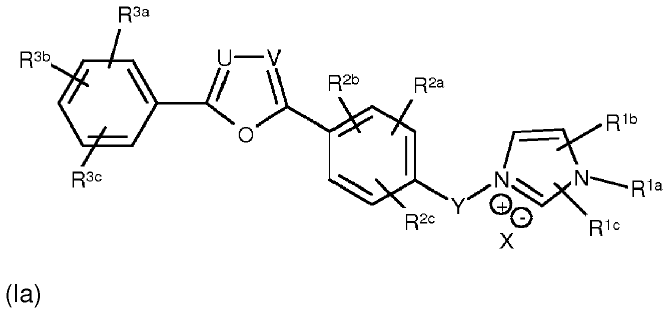 wherein:
R^{1a} is a C₁-C₃₀ alkyl group;
R^{1b} and R^{1c} are H; 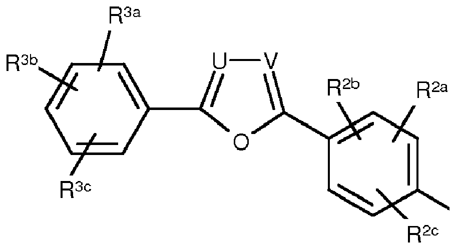 is a fluorophore group wherein:
R^{2a}, R^{2b}, R^{2c}, R^{3a}, R^{3b}, R^{3c} are, at each occurrence, independently selected from among H, C₁-C₃₀ alkyl groups, and C₃-C₇ cycloalkyl groups;
Y is a C₁-C₆ alkylene group,
U, V represent independently CR²⁶ or N, it being understood that at least one of U or V represents N;
R²⁶ is H, a C₁-C₆ alkyl group, OR²², NR²³R²⁴, NO₂, C(=O)R²², CO₂R²², OC(=O)R²², C(=O)NR²³R²⁴, OC(=O)NR²³R²⁴, SO₃H, PO₄H, CN;
R²² is, at each occurrence, independently selected from among H, a C₁-C₆ alkyl group, a C₆-C₁₀ aryl group and aralkyl group;
R²³ and R²⁴ are, at each occurrence, independently selected from among H, a C₁-C₆ alkyl group, a C₆-C₁₀ aryl group, or else R²³ and R²⁴ form with the nitrogen atom to which they are attached a heterocyclic group with 3 to 7 members;
X- is an organic or inorganic anion.

2. The compound according to claim 1, wherein R^{1a} is a C₁-C₁₆ alkyl group.

3. The compound according to any of claims 1 to 2, wherein U is CR²⁶.

4. The compound according to any of claims 1 to 2, wherein U is CH.

5. The compound according to any of claims 1 to 4, wherein Y is a CH₂ group.

6. The compound according to any of claims 1 to 5, wherein X- is an anion selected from among a halide, P(R⁴)₆⁻, B(R⁴)₄⁻, SCN⁻, (R⁵SO₂)₂N⁻, R⁵OSO₃, R⁵SO₃⁻, carborane, carbonate (CO₃²⁻), hydrogencarbonate (HCO₃⁻), alcoholate (R⁴O⁻), carboxylate (R⁴COO⁻), amide (NH₂⁻), phosphate (PO₄⁻), SiF₆⁻, SbF₆⁻, I₃⁻, nitrate (NO₃⁻), halide oxide, silcate, sulfate (SO₄⁻), sulfonate (R⁴SO₃⁻), cyanide (CN⁻), carbanions, or metallate; wherein:
R⁴ is, at each occurrence, a group independently selected from among a C₁-C₆ alkyl group, a C₆-C₁₀ aryl group, an aralkyl group;
R⁵ is, at each occurrence, independently selected from among a C₁-C₂₀ alkyl group, a C₁-C₂₀ halogenoalkyl group, a C₆-C₁₀ aryl group, aralkyl group.

7. The compound according to any of claims 1 to 6, wherein X- is selected from among Br⁻, PF₆⁻, BF₄⁻, (CF₃SO₂)₂N⁻, C₁₂H₂₅OSO₃⁻, C₁₆H₃₃OSO₃⁻, CF₃SO₃⁻.

8. The compound according to claim 1, wherein the compound of formula (Ia) is selected from among:
- 1-methyl-3-[4-(5-phenyl-oxazol-2-yl)-benzyl]-3H-imidazol-1-ium bromide
- 1-hexyl-3-[4-(5-phenyl-oxazol-2-yl)-benzyl]-3H-imidazol-1-ium bromide
- 1-octyl-3-[4-(5-phenyl-oxazol-2-yl)-benzyl]-3H-imidazol-1-ium bromide
- 1-decyl-3-[4-(5-phenyl-oxazol-2-yl)-benzyl]-3H-imidazol-1-ium bromide
- 1-dodecyl-3-[4-(5-phenyl-oxazol-2-yl)-benzyl]-3H-imidazol-1-ium bromide
- 3-[4-(5-phenyl-oxazol-2-yl)-benzyl]-1-tetradecyl-3H-imidazol-1-ium bromide
- 1-hexadecyl-3-[4-(5-phenyl-oxazol-2-yl)-benzyl]-3H-imidazol-1-ium bromide
- 1-hexadecyl-3-[4-(5-phenyl-oxazol-2-yl)-benzyl]-3H-imidazol-1-ium hexafluorophosphate
- 1-hexadecyl-3-[4-(5-phenyl-oxazol-2-yl)-benzyl]-3H-imidazol-1-ium bis(trifluoromethylsulfonyl)imide
- 1-hexadecyl-3-[4-(5-phenyl-oxazol-2-yl)-benzyl]-3H-imidazol-1-ium dodecylsulfate
- 1-hexadecyl-3-[4-(5-phenyl-oxazol-2-yl)-benzyl]-3H-imidazol-1-ium hexadecylsulfate.

9. The use for detecting gamma, X, neutron, proton radiations, of a fluorophore compound as defined according to any of claims 1 to 8.

10. The use of a compound as defined according to any of claims 1 to 8, for making a radiodetection, industrial or medical dosimetry instrument.

11. The use of a fluorophore compound as defined according to any of claims 1 to 8, for discrimination of proton/gamma, proton/X, neutron/gamma, neutron/X, alpha/gamma, alpha/X radiations.

12. A material comprising a compound according to any of claims 1 to 8.

13. The material according to claim 12, **characterized in that** the material is a transparent plastic material.

14. A method for preparing a compound of formula (Ia) as defined according to any of claims 1 to 8, said method comprising the reaction of a compound of formula (II) with a compound of formula (III): 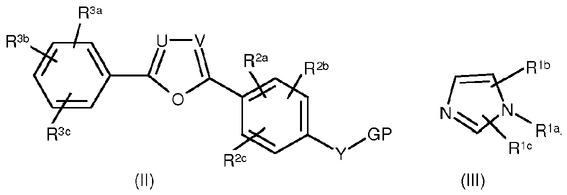 wherein R^{1a}, R^{1b}, R^{1c}, R^{2a}, R^{2b}, R^{2c}, R^{3a}, R^{3b}, R^{3c}, U and Y are as defined according to any of claims 1 to 8, and GP represents a leaving group.

15. The method according to claim 14, **characterized in that** the reaction is carried out in an aprotic polar solvent.
